# EUROPEAN PATENT APPLICATION

(11) **EP 4 759 300 A1**
(43) Date of publication of application: **17.06.2026**
(21) Application number: 24383371.2
(22) Date of filing: 13.12.2024
(51) Int. Cl.: A61K 9/00, A61K 9/50, A61K 35/28, A61P 9/10, C12N 5/0797, C12N 15/113

(54) **LIPID NANOPARTICLES AND THEIR USE IN THE TREATMENT OF ISCHEMIC HEART DISEASES**

(71) Applicant: Universitat de València, 46010 Valencia (ES); Fundacion para la Investigacion del Hospital Clinico de la Comunidad Valenciana, 46010 Valencia (ES); Fundación Para la Investigación del Hospital Universitario y Politécnico La Fe de la Comunidad Valenciana, 46026 Valencia (ES)
(72) Inventor: RÍOS NAVARRO, César, 46010 VALENCIA (ES); BUIGUES CARAVACA, Marc, 46026 VALENCIA (ES); GÓMEZ FERRER, Marta, 46026 VALENCIA (ES); ONTORIA OVIEDO, Imelda, 46026 VALENCIA (ES); PEIRÓ MOLINA, Esteban, 46026 VALENCIA (ES); SEPÚLVEDA SANCHIS, Pilar, 46026 VALENCIA (ES); RUIZ SAURI, Amparo, 46010 VALENCIA (ES); BODÍ PERIS, Vicente, 46010 VALENCIA (ES)
(74) Representative: Herrero & Asociados, S.L.

(57) **Abstract**

The present invention refers to lipid-based nanoparticles characterized by specific proteins and miRNAs that confer therapeutic capacity to these nanoparticles. The present invention also refers to the clinical applications of these nanoparticles for the treatment of ischemic heart diseases. Pharmaceutical compositions comprising one or more of these lipid nanoparticles are also contemplated. Finally, the use of the nanoparticles as a nanocarriers, nanovectors and/or nanocapsules for the release and/or encapsulation of molecules is also referred.

## Description

### Field of the invention

The present invention relates generally to the Health Sector, in particular the field of Tissue Regeneration. Particularly, the invention relates to lipid nanoparticles and lipid formulations comprises said nanoparticles, and their use in the regeneration of cardiac tissue.

### Background of the invention

Coronary artery disease (CAD), also known as ischemic heart disease, is the most prevalent type of cardiovascular disease (CVD) and the leading cause of mortality and morbidity worldwide (Roth, G.A. et al. Global, Regional, and National Age-Sex-Specific Mortality for 282 Causes of Death in 195 Countries and Territories, 1980-2017: A Systematic Analysis for the Global Burden of Disease Study 2017. Lancet 2018, 392, 1736-1788, doi:10.1016/S0140-6736(18)32203-7*).* CAD is characterized by an imbalance between the supply and demand of oxygen to the cardiac tissue that triggers a series of events ranging from necroptosis of cardiomyocytes (CM) first, to fibrosis and adverse cardiac remodelling, which can occur time after an acute ischemic event. These alterations, both physiological and structural, can lead to the progressive deterioration of cardiac function and, finally, to the onset of heart failure (HF) *(*Opie, L.H.; et al. Controversies in Ventricular Remodelling. Lancet 2006, 367, 356-367, doi:10.1016/S0140-6736(06)68074-4*).* Considering the ethiology and pathogenesis of coronary artery occlusion, acute myocardial infarction (AMI) can be classified into five types *(*Thygesen, K. et al. Fourth Universal Definition of Myocardial Infarction (2018). Circulation 2018, 138, e618-e651, doi:10.1161/CIR.0000000000000617*;* Hartikainen, T.S. et al. Clinical Application of the 4th Universal Definition of Myocardial Infarction. Eur Heart J 2020, 41, 2209-2216, doi:10.1093/EURHEARTJ/EHAA035). Among them, in Type 1 MI a complete or parcial occlusion of the coronary artery occurs due to a plaque rupture and detachment, whereas in Type 2 MI, the mismatch between oxygen supply and demand in the myocardium, typically without plaque rupture or acute coronary thrombosis. Unlike type 1 MI, which is caused by an acute coronary event such as plaque rupture, type 2 MI results from conditions that place additional strain on the heart, such as severe hypertension, arrhythmias, or hypoxia, leading to myocardial ischemia. In murine models, isoproterenol administration induces significant myocardial injury and necrosis by overstimulating the heart and triggering a supply-demand mismatch in oxygen, which is characteristic of type 2 MI. This method has been widely used to simulate the effects of myocardial stress that occur in human patients suffering from non-coronary-related myocardial ischemia (RONA G, et al. An infarct-like myocardial lesion and other toxic manifestations produced by isoproterenol in the rat. Arch Pathol (Report missing IFs). 1959 Apr;67(4):443-55*).*

Small extracellular vesicles (sEVs) derived from mesenchymal stromal cells (MSCs) have been proposed as a promising therapeutic option for the treatment of CAD due to their role in cardiac regeneration. It has been proven that these sEVs can recapitulate the beneficial effects of MSCs, such as promoting angiogenesis, reducing apoptosis, modulating the immune system, and reducing the fibrosis process (Sun, S.J.; Wei, R.; Li, F.; Liao, S.Y.; Tse, H.F. Mesenchymal Stromal Cell-Derived Exosomes in Cardiac Regeneration and Repair. Stem Cell Reports 2021, 16, 1662-1673*).* Furthermore, the use of sEVs offers additional advantages compared to the use of MSCs, such as lower immunogenicity, greater ease of production and storage, and a more affordable cost, in addition to not being a live product. However, clinical application MSC-derived sEVs is hindered by batch-to-batch variability from primary cultures, limited scalability of production and reduced therapeutic potential in humans in comparison with animal models. Thus, the challenge of current research is to improve the therapeutic efficacy of sEVs in order to make EV-based therapy feasible. These strategies aim to develop less heterogeneous sEV-based biological products with consistent and improved therapeutic performance to jump into clinical arena.

HIF1α is a transcription factor that stabilises under ischaemic conditions and induces VEGF expression (Tsai, C.C.; et al. Hypoxia Inhibits Senescence and Maintains Mesenchymal Stem Cell Properties through Down-Regulation of E2A-P21 by HIF-TWIST. Blood 2011, 117, 459-469, doi:10.1182/BLOOD-2010-05-287508*).* It has been showed that sEVs from human telomerase (hTERT)-immortalized MSC overexpressing HIF1α (sEVsTH) exerted increased pro-angiogenic and immunomodulatory properties (Gómez-Ferrer, M. et al. A. HIF1α and Pro-Inflammatory Signaling Improves the Immunomodulatory Activity of MSC-Derived Extracellular Vesicles. Int J Mol Sci 2021, 22, doi:10.3390/IJMS22073416*;* Gómez-Ferrer, M.et al. HIF-Overexpression and Pro-Inflammatory Priming in Human Mesenchymal Stromal Cells Improves the Healing Properties of Extracellular Vesicles in Experimental Crohn's Disease. Int J Mol Sci 2021, 22, doi:10.3390/IJMS222011269*).*

In addition, Notch signalling pathway is highly relevant in mammalian embryonic development and maintains its importance over time by participating in intercellular communication. Notch1 is a receptor with an intracellular and an extracellular domain. The binding of Notch receptor to its ligands results in the cleavage of the intracellular domain (N1ICD), which translocates to the nucleus and activates transcription of several target genes acting as a transcription factor *(*Zhang, R.; et al. In Vivo Cardiac Reprogramming Contributes to Zebrafish Heart Regeneration. Nature 2013, 498, 497, doi:10.1038/NATURE12322). N1ICD is crucial in the regulation of cardiogenesis through different elements of the pathway, such as HEY1 and HEY2, which are downstream genes involved in endocardial differentiation (Siebel, C.; Lendahl, U. Notch Signaling in Development, Tissue Homeostasis, and Disease. Physiol Rev 2017, 97, 1235-1294, doi:10.1152/PHYSREV.00005.2017). Abnormalities in the Notch pathway can lead to congenital malformations of the heart (Zhou, X.L.; Liu, J.C. Role of Notch Signaling in the Mammalian. Brazilian Journal of Medical and Biological Research 2014, 47, 1, doi:10.1590/1414-431X20133177*;* Wang, Y. et al. NOTCH Signaling in Aortic Valve Development and Calcific Aortic Valve Disease. Front Cardiovasc Med 2021, 8, 682298, doi:10.3389/FCVM.2021.682298*).* Due to its involvement in developmental and differentiation processes, the Notch signalling pathway is also of special interest in the study of the ischaemic heart and cardiac regeneration, and in processes of angiogenesis and fibrosis (Gonzalez-King, H.; et al. Hypoxia Inducible Factor-1α Potentiates Jagged 1-Mediated Angiogenesis by Mesenchymal Stem Cell-Derived Exosomes. Stem Cells 2017, 35, 1747-1759, doi:10.1002/stem.2618*;* Nistri, S. et al. Notch Signaling in Ischemic Damage and Fibrosis: Evidence and Clues from the Heart. Front Pharmacol 2017, 8, 260676, doi:10.3389/FPHAR.2017.00187/BIBTEX). Even though this receptor is not active in adult cardiac cells, this pathway is reactivated after reperfusion injury following AMI (Gude, N.A.; et al. Activation of Notch-Mediated Protective Signaling in the Myocardium. Circ Res 2008, 102, 1025-1035, doi:10.1161/CIRCRESAHA.107.164749*).* Modulation of Notch pathway has also been used to increase angiogenesis or to reduce fibrosis, through antagonyzing the TGFβ pathway refer.

It has been showed a direct link between the HIF1α and Notch pathways in MSCs through SUMOylation of N1ICD. Overexpression of HIF1α in MSCs (MSCs-H) led to increased expression of several Notch ligands (such as Jagged1, DII1, DII3, and DII4), thus enhancing Notch signaling. MSC-H displayed significantly higher activation of Notch downstream target genes (Hes1, Hey1, Hey2) compared to control cells, especially when the cells were exposed to Jagged1.

In addition, MSC-HIF displayed higher levels of SUMOylated N1ICD, which facilitated its nuclear translocation and transcriptional activity. This process was critical for the observed increase in MSC migration and invasiveness, but it did not affect cell proliferation, which was shown to be Notch-independent (Ciria M. et al. Mesenchymal Stem Cell Migration and Proliferation Are Mediated by Hypoxia-Inducible Factor-1α Upstream of Notch and SUMO Pathways. Stem Cells Dev. 2017 Jul 1;26(13):973-985. doi:10.1089/scd.2016.0331. Epub 2017 May 18. PMID: 28520516*).*

The authors of the present invention have used an hTERT-immortalized MSC line (MSC-T) to overexpress HIF1α and N1ICD and to generate a boosted MSC-THN cell line in order to enhance the therapeutic capacity of their released sEVs. The results demonstrate a higher therapeutic efficacy of MSC-THN-derived sEVs in vitro and in murine models of MI type 1 and type 2 in terms of increased angiogenesis, reduction of cardiac oxidative stress, apoptosis, fibrosis and hyperthrophy. Moreover, MSC-THN-derived sEVs (EVs-THN) were highly effective in reducing fibroblast activation, thus modulating myofibroblasts expression in the infarcted region and improving cardiac function as assessed by echocardiography. The therapeutic effects of EVS-THN were further corroborated in a swine model of ischemia/reperfusion injury.

However, it is necessary to characterize EVs in order to identify the molecules responsible for their regenerative capacity. This characterization is essential for understanding the mechanisms of action of these vesicles and for translating their therapeutic benefits into clinically applicable solutions.

The author of the present invention, after a huge experimental effort, have characterized the sEVs derived from MSCs modified to overexpress HIF1α and N1ICD, identifying the specific proteins and miRNAs that confer therapeutic capacity to these sEVs. These important findings enable the design and synthesis of optimized nanoparticles that incorporate these therapeutic components, bypassing the inherent variability and scalability issues associated with biologically derived sEVs.

The ability to engineer synthetic nanoparticles mimicking the protein and miRNA profiles of MSC-derived sEVs significantly broadens the scope of therapeutic applications. These nanoparticles can be tailored for specific therapeutic outcomes, offering a scalable, reproducible, and cost-effective alternative to natural sEVs while maintaining their regenerative and reparative potential. Moreover, the synthetic approach allows for consistent quality control, improved shelf stability, and the possibility of targeting additional pathways through precise molecular engineering.

### Brief description of the drawings

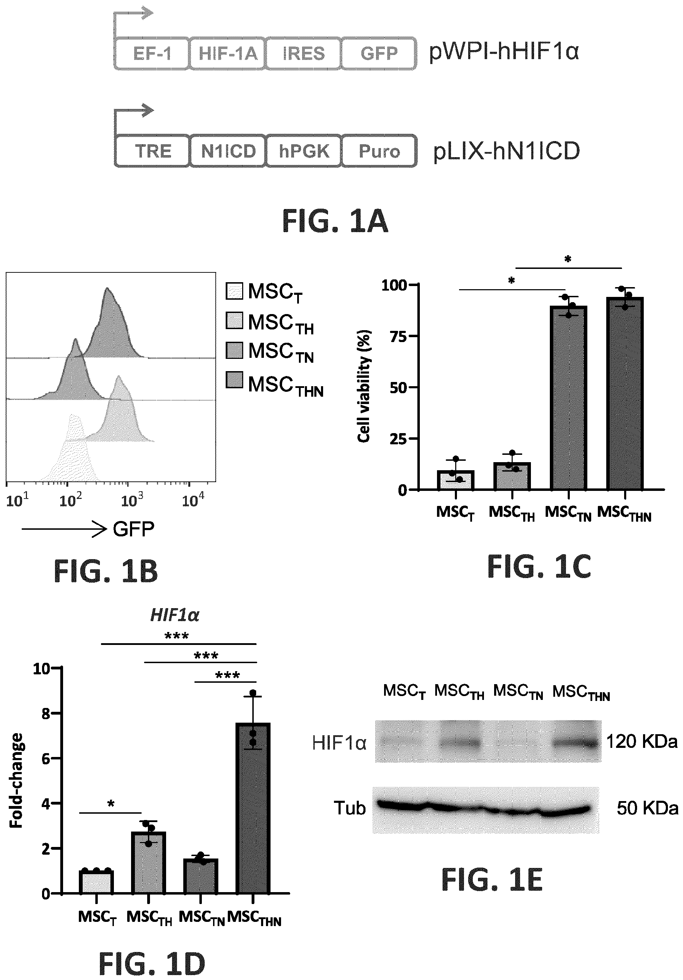

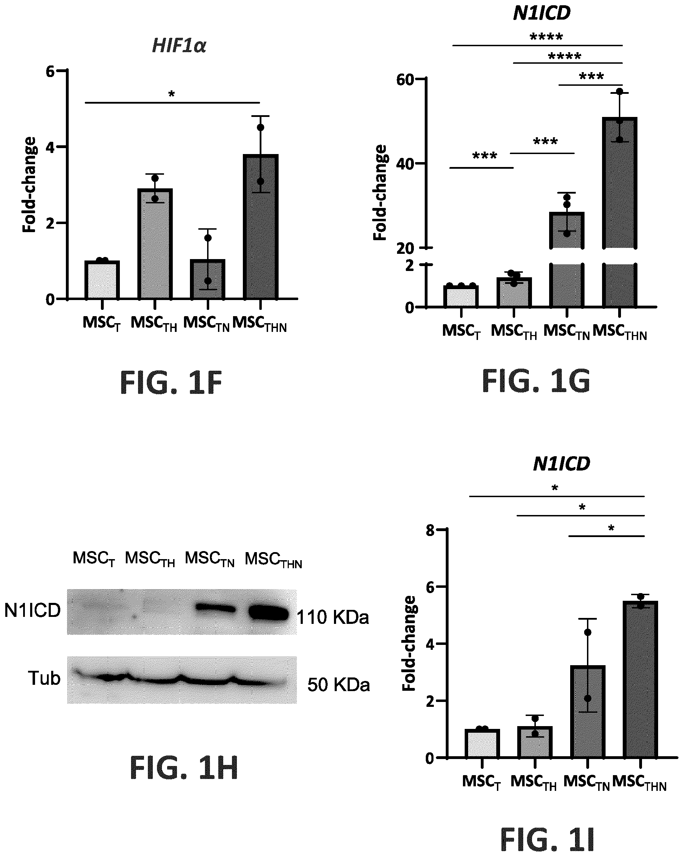

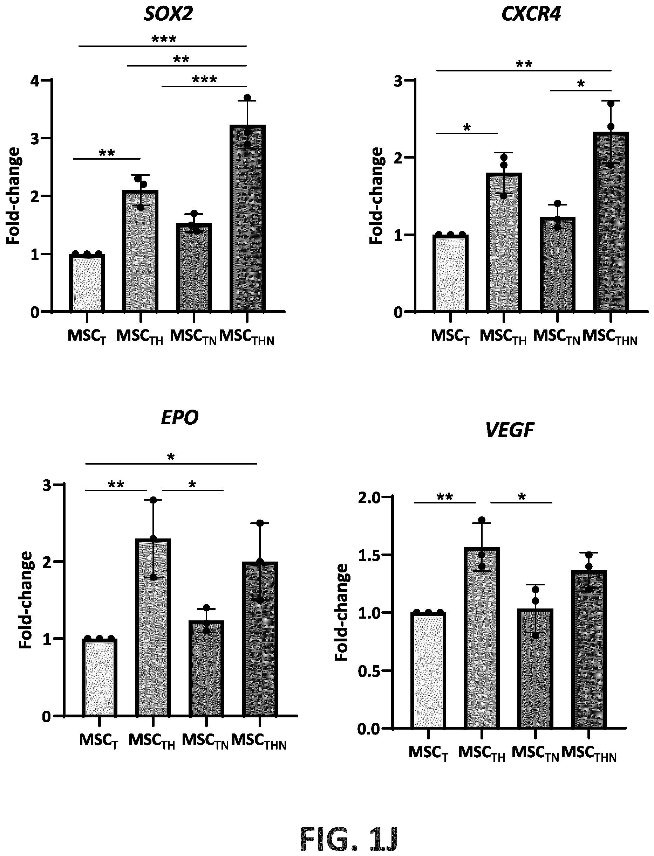

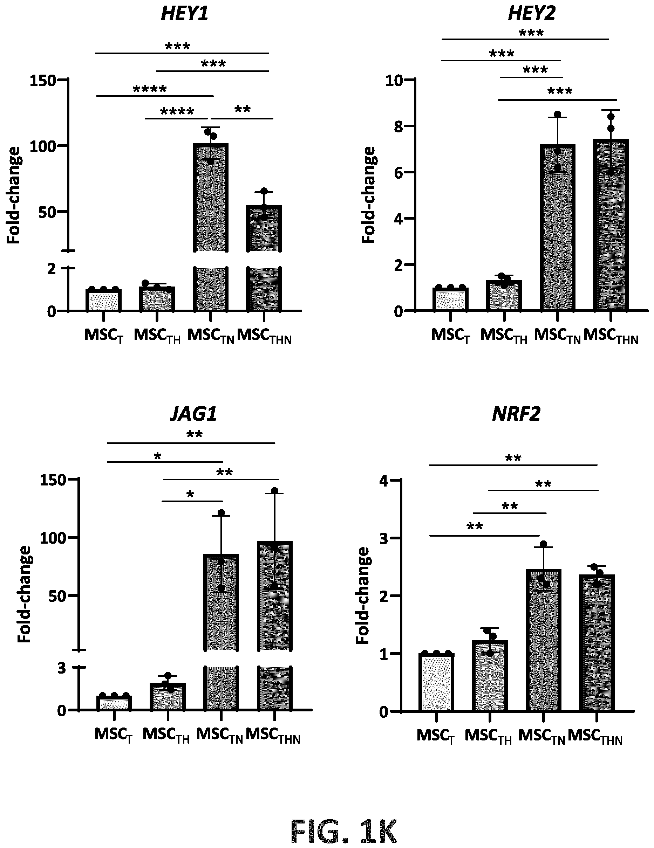

**Figure 1****. Expression of genes of interest in the generated MSC lines.** A) Schematic representation of the workflow for the generation of MSC-TH, MSC-TN and MSC-THN cell lines. B) Histogram of flow cytometry analysis of GFP+ cells. C) Quantification of live MSC detected by the MTT assay after being treated or not with puromycin for 48 h. D) mRNA expression levels of HIF1 *α* in the different cell lines. E) Expression of HIF1 *α* measured by western blot analysis. Tubulin was used as load control. F) Quantification of HIF1 *α* in MSC was obtained with densitometric analysis and normalized with tubulin. G) mRNA expression levels N1ICD in the different cell lines. H) Expression of N1ICD measured by western blot analysis. Tubulin was used as load control. I) Quantification of N1ICD in MSC was obtained with densitometric analysis and normalized with tubulin. J) Expression of HIF1 *α* target genes SOX2, CxCR4, EPO and VEGF in the different cell lines. K) Expression of N1ICD downstream genes HEY1, HEY2 and NRF2 and the Notch ligand JAG1 in the different MSC lines. Expression is shown as mean ± SD normalized against MSC-T cells. Statistical analysis was performed by ANOVA followed by Tukey's post-hoc test. * p < 0.05, ** p < 0.01 and *** p < 0.001.

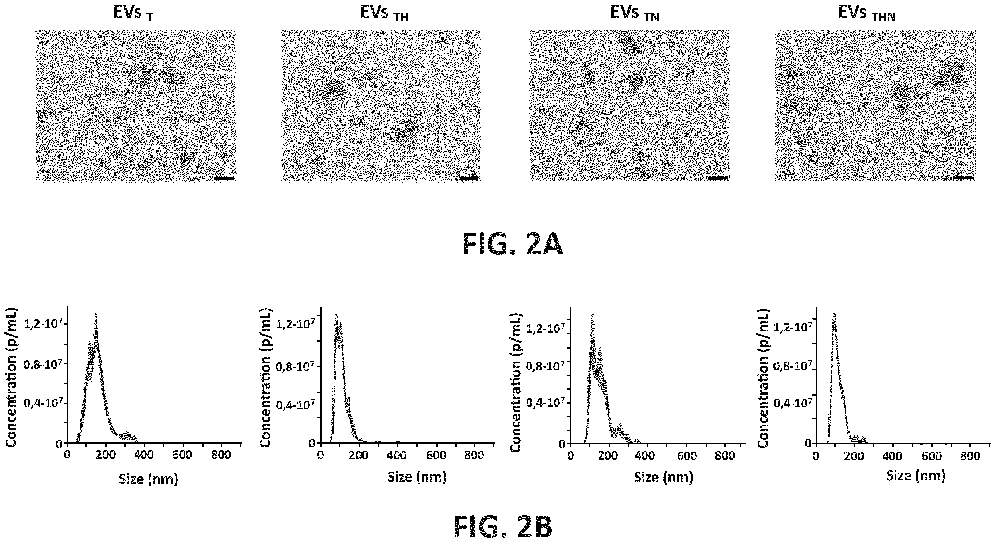

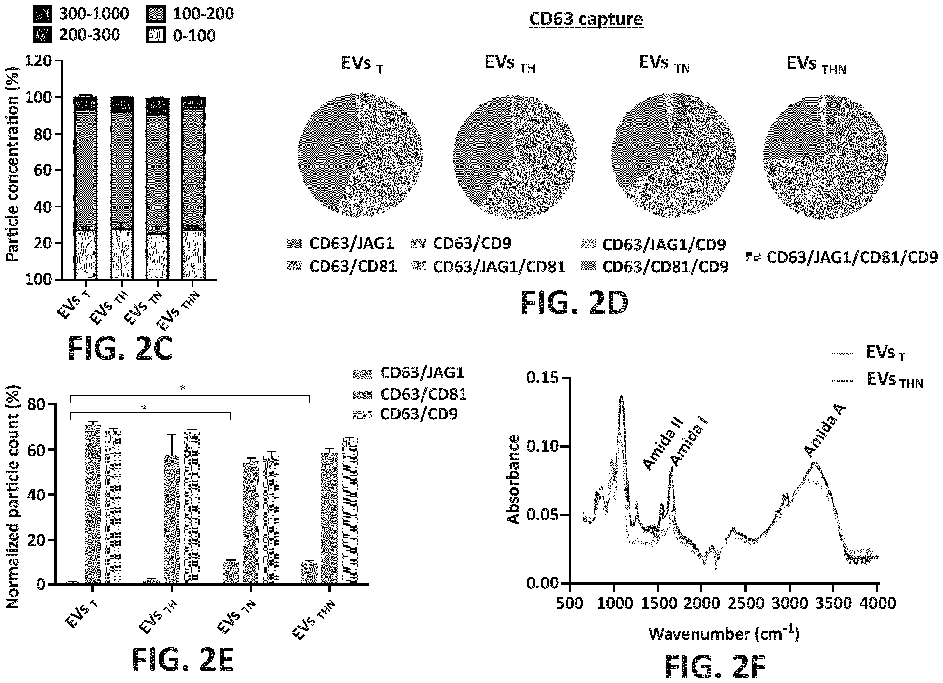

**Figure 2****. Characterisation of sEVs.** A) Representative images of sEVs obtained by transmission electron microscopy (scale bar = 100 nm). (B,C) NTA result showing the average size of sEVs (nm) and EV size distribution histograms of sEVs. D) Pie charts showing the co-expression of the different proteins (JAG1, CD81 and CD9) in sEVs captured with CD63. E) ExoView analysis showing the percentage of sEVs positive for JAG1, CD81 or CD9 in isolates from the different MSC lines, after capture with CD63. F) Molecular profile and functional groups of sEVs-T and sEVs-THN measured by ATR-FTIR.

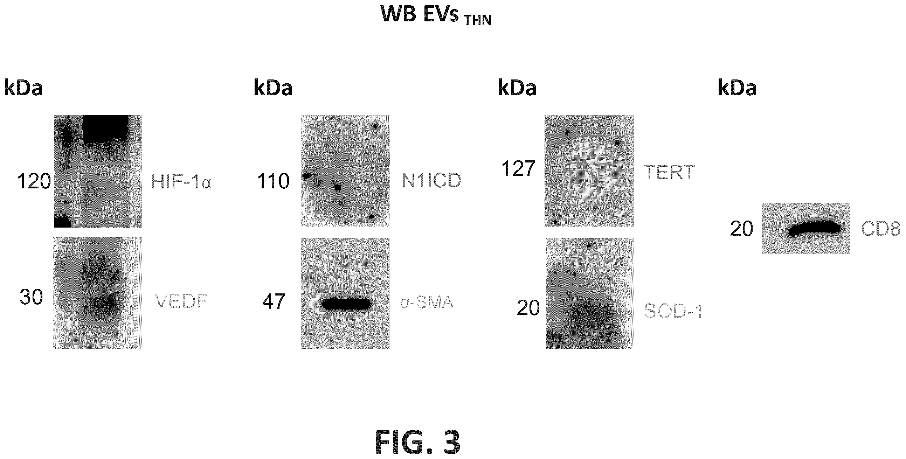

**Figure 3****. Characterization of sEVs.** Representative western blotting of HIF1 *α*, N1ICD, TERT, VEGF, *α-*SMA and SOD-1 protein expression. CD81 protein expression was used as a positive control for sEVs.

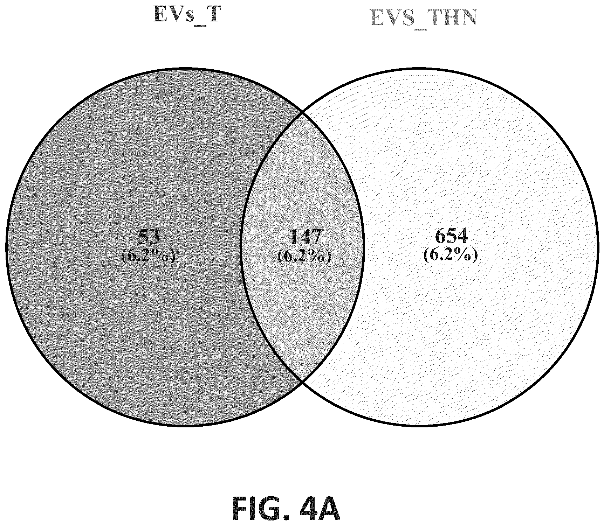

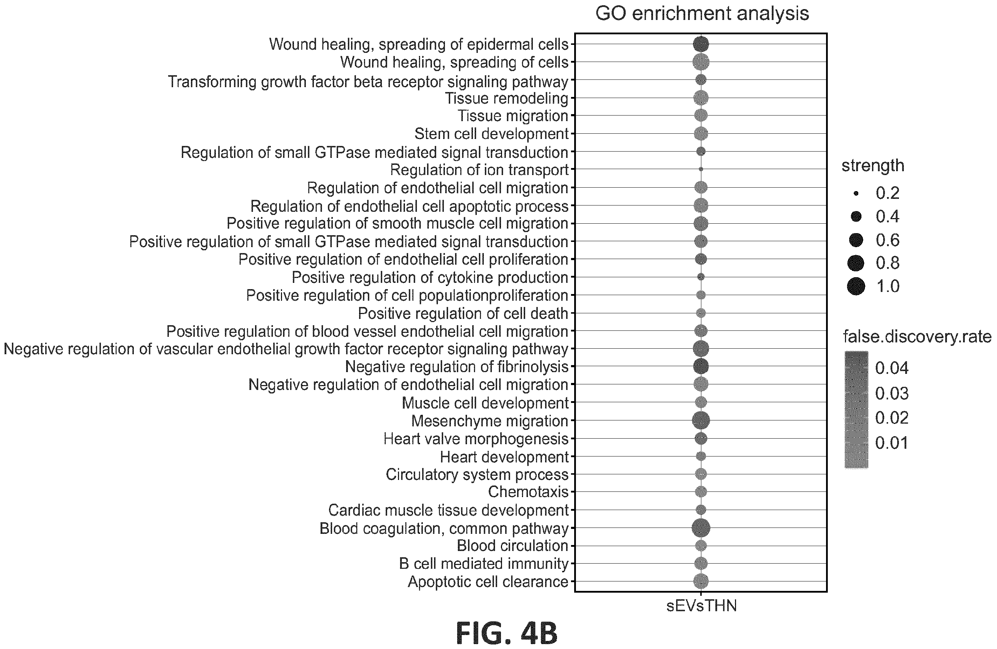

**Figure 4****. Graphical representation of proteomic analysis of sEVsT and sEVsTHN.** A) Venn diagram of the proteins identified in MSC-derived EVs-T and EVs-THN after proteomic analysis. B) Graphical representation of biological processes related to cardiac tissue repair and regeneration that were detected exclusively with the proteins identified in EVs-THN.

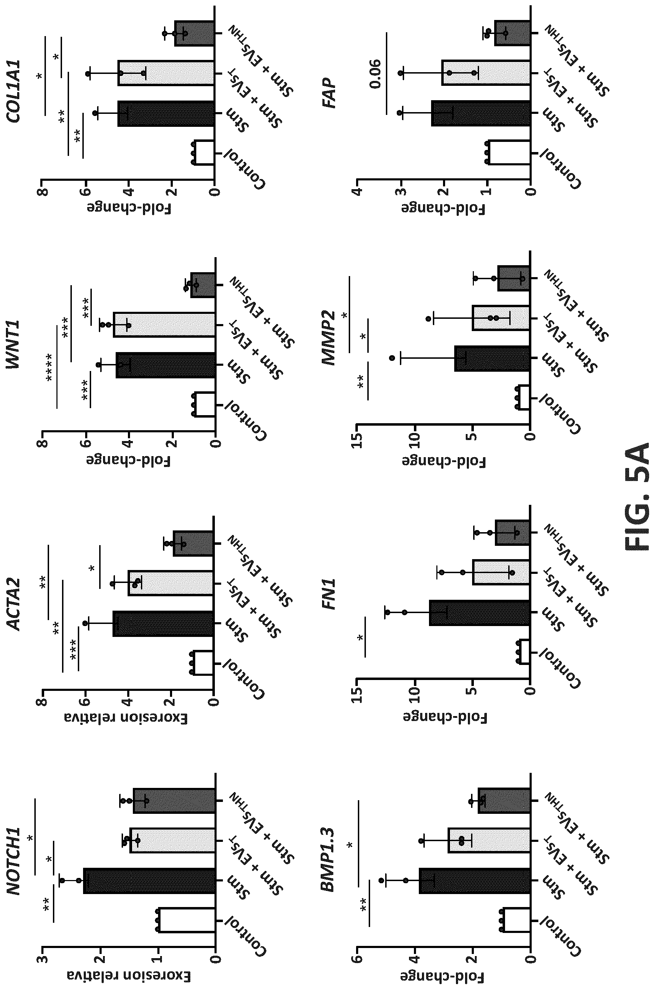

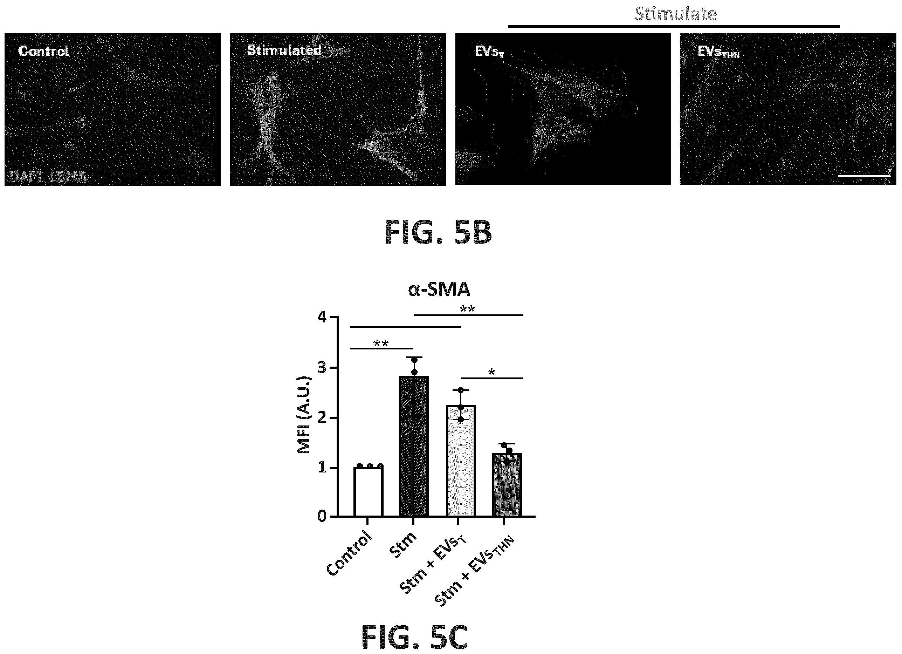

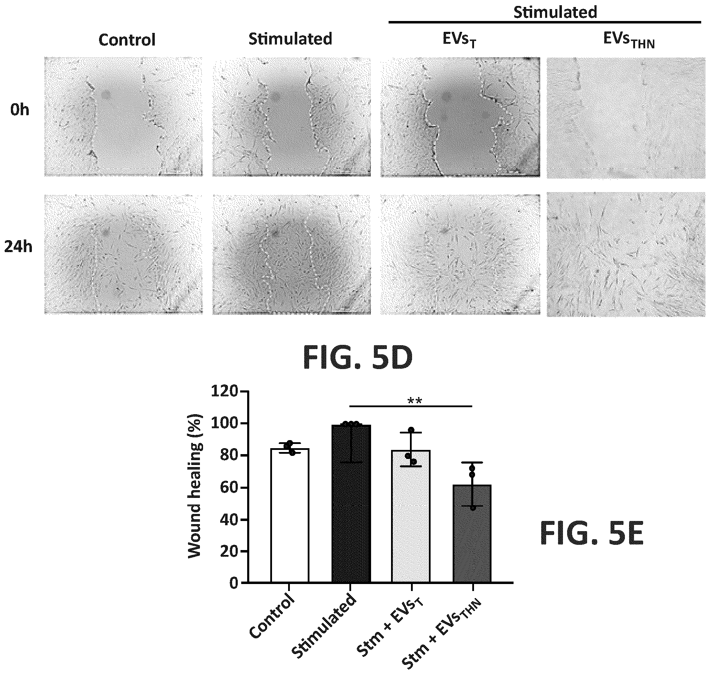

**Figure 5****. sEVsTHN reduce fibrosis markers and migration in stimulated HCF.** A) Expression of profibrotic genes after stimulation with the pro-fibrotic cocktail (dextran sulphate, ascorbic acid 2-phosphate, TGF *β*1) for 24 hours, with or without sEVs. B) Representative immunofluorescence images of *α* SMA in HCF after stimulation with the profibrotic cocktail for 24 hours, with or without sEVs. Scale bar = 50 µm C) Quantification of mean fluorescence intensity. D) Representative images of the wound closure assay in HCF after stimulation with a pro-fibrotic cocktail. E) Quantification of migration by measuring the percentage closure at 24 hours, calculated as area occupied by cells/initial wound area. Results are shown as mean ± SD normalised against the Control condition (unstimulated fibroblasts). Statistical analysis was performed by ANOVA followed by Tukey's post-hoc test. * p < 0.05, ** p < 0.01 and *** p < 0.001.

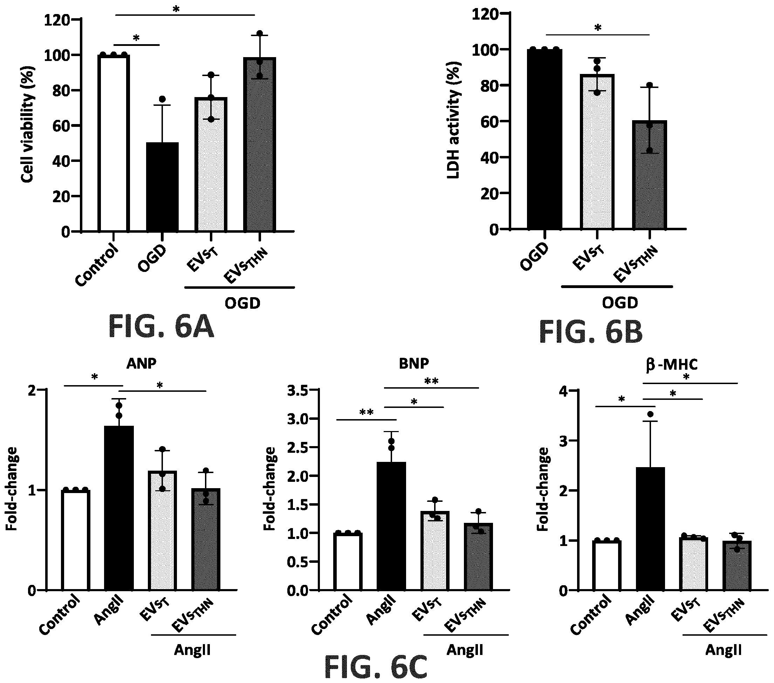

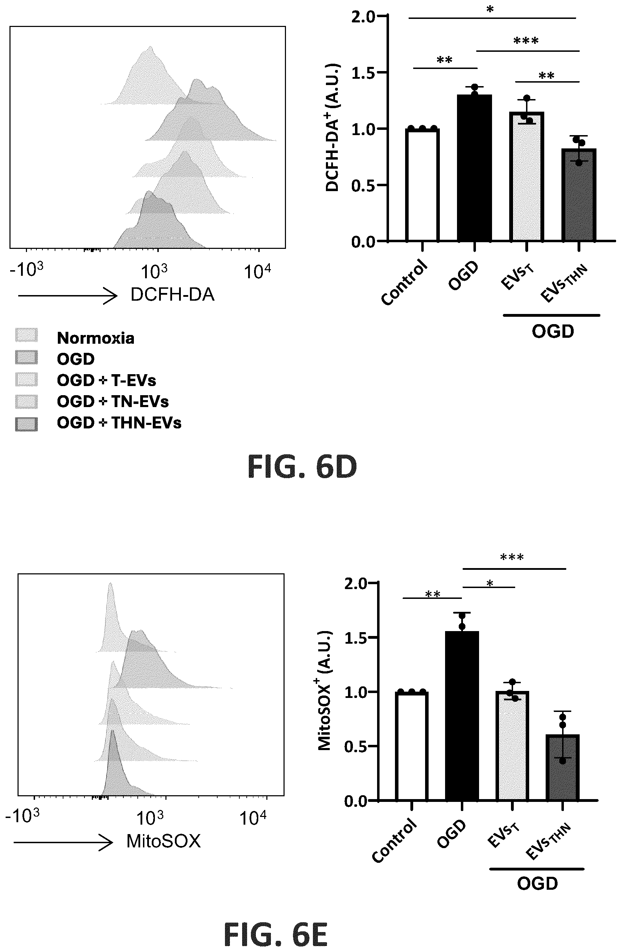

**Figure 6****. sEVs-THN increase viability, reduce ROS and reduce hypertrophy markers in H9C2 cells.** A) Cell viability measured by CCK-8 assay in H9C2 cells after 7 hours of OGD with/without sEVs. B) Measurement of LDH activity to indirectly assess plasma membrane integrity of H9C2 cells after 7 hours of OGD with/without sEVs. C) Expression level of hypertrophy markers. The graphs show the relative expression of the main hypertrophy-related genes in cardiomyocytes: atrial natriuretic peptide (ANP), brain natriuretic peptide (BNP) and βmyosin heavy chain (βMHC), after exposure to angiotensin II (AngII, hypertrophic stimulus). D) Representative flow cytometry histograms of H9C2 cells stained with DCFH-DA after 7 hours of OGD. DCFH-DA (intracellular ROS) values in H9C2 cells. E) Representative flow cytometry histograms of H9C2 cells stained with MitoSOX after 7 hours of OGD. MitoSOX (mitochondrial superoxide) values in H9C2 cells. Graphs show mean ± SD normalised against normoxic condition. Statistical analysis was performed by ANOVA followed by Tukey's post-hoc test. * P < 0.05, ** P < 0.01 and *** P < 0.001 vs OGD/Control condition.

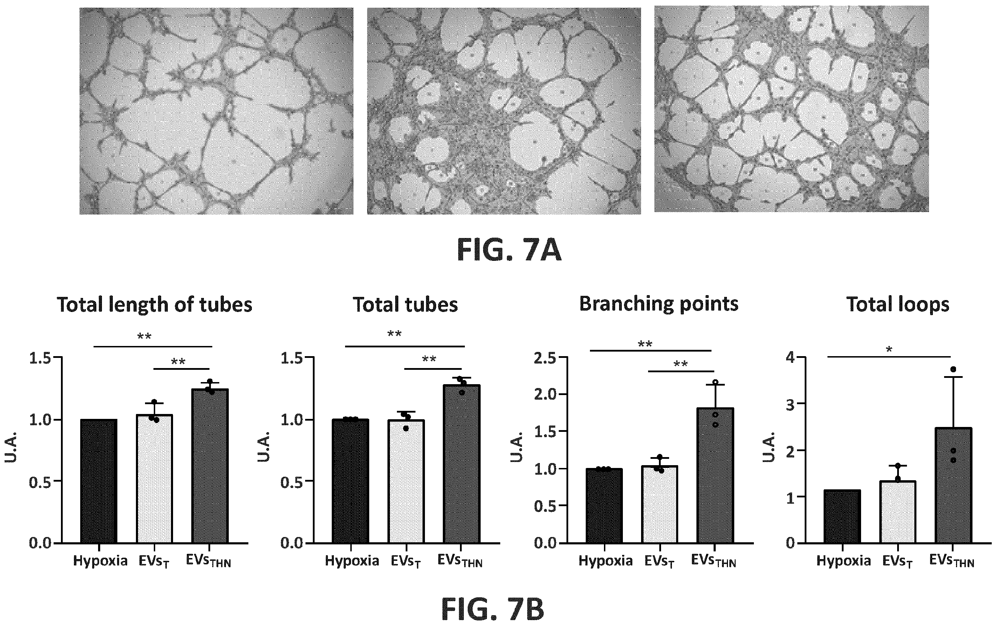

**Figure 7****. sEVs-THN promote tube formation in endothelial cells in vitro.** A) Representative images of the tube formation assay on Matrigel using HUVEC cells under hypoxia. B) Quantification of parameters related to angiogenic potential: total tube length, total tubes, total branching points and total loops. Graphs show mean ± SD values normalised against the control condition (hypoxia). Statistical analysis was performed by ANOVA followed by Tukey's post-hoc test. * p < 0.05, ** p < 0.01 and *** p < 0.001.

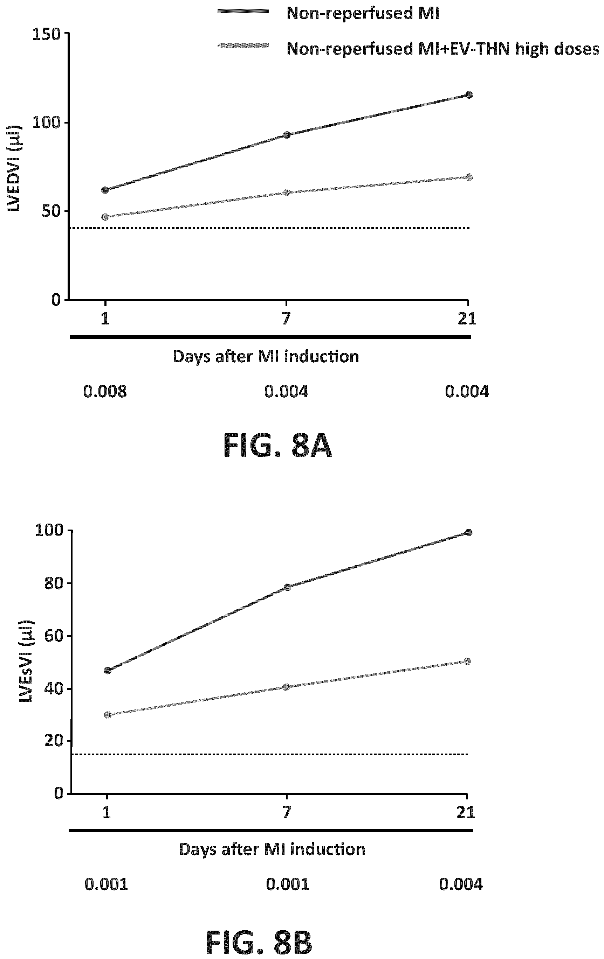

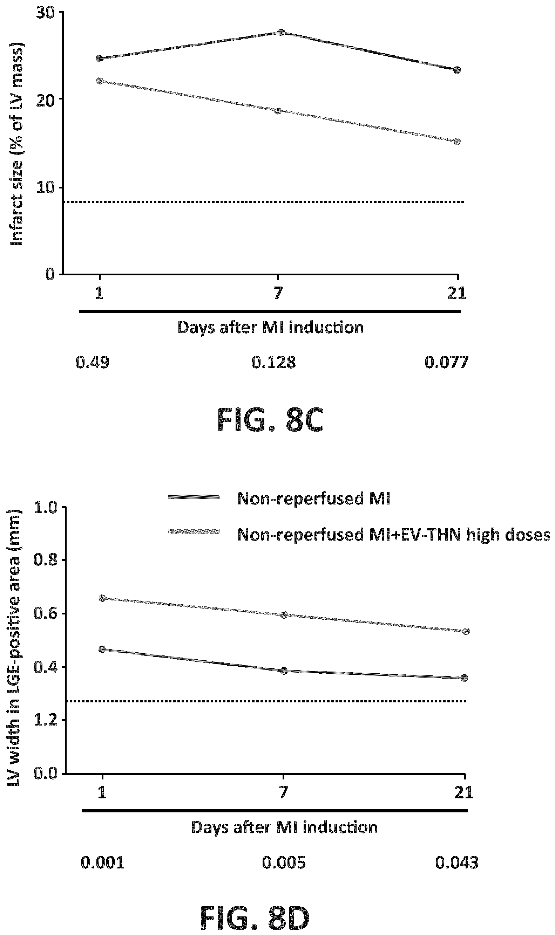

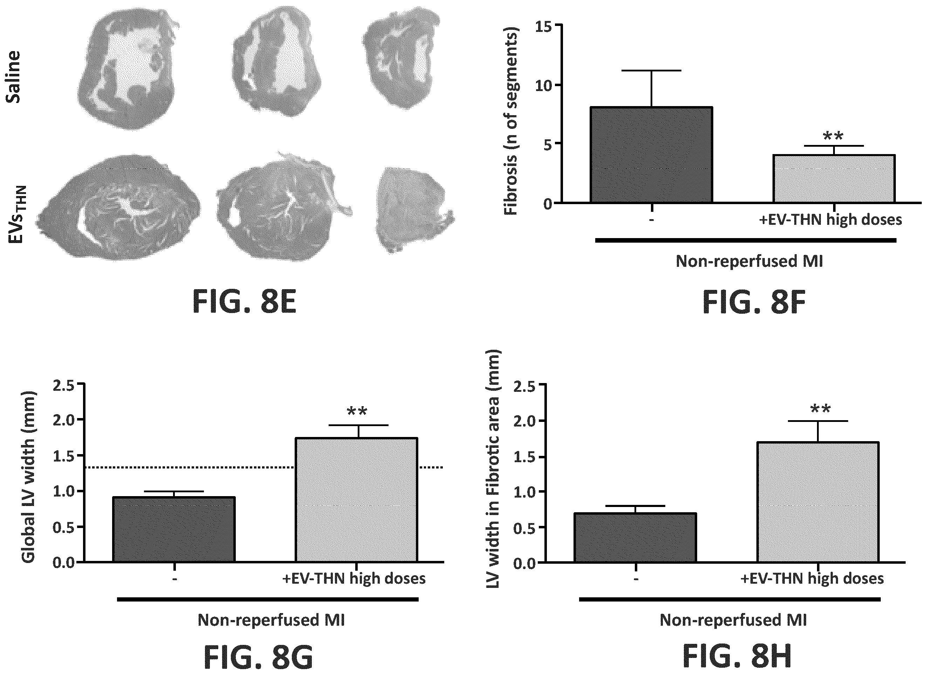

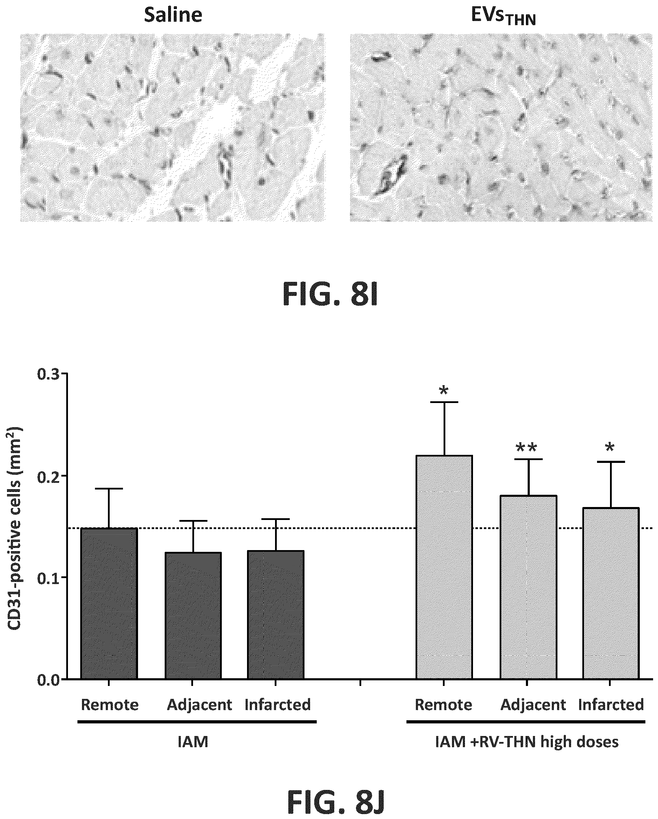

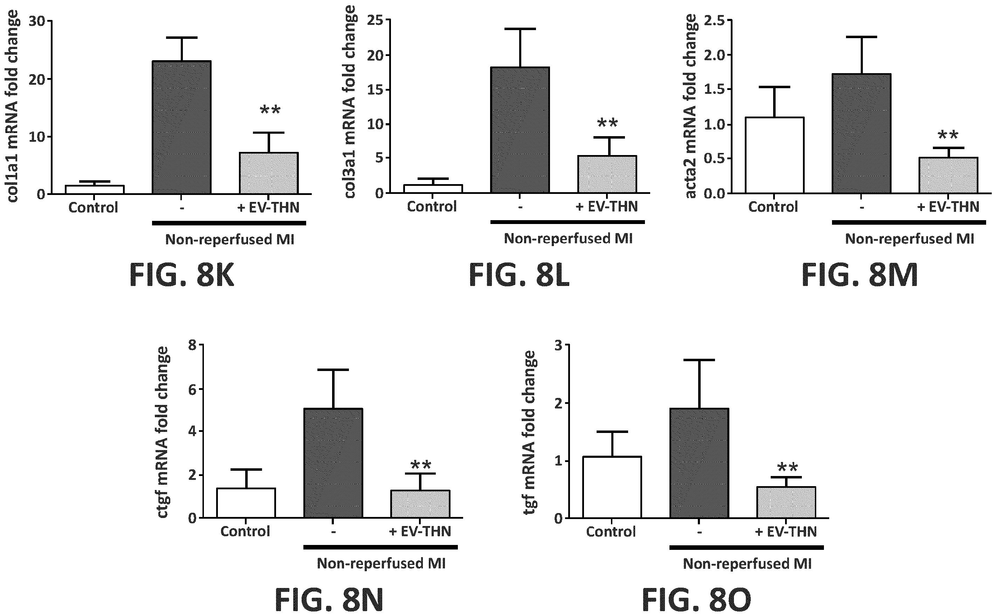

**Figure 8****. sEVs-THN reduces cardiac adverse remodelling and interstitial fibrosis and enhances angiogenesis in a mice model of myocardial ischemia.** Left ventricular end-diastolic (A) and end-systolic (B) volume indices together with infarct size (C) and left ventricular width in infarcted areas (D) were determined in cardiac magnetic resonance studies performed at 1, 7, and 21 days after infarct induction. (E) Representative images from Masson's trichrome staining from hearts submitted to sEVs-THN treatment or saline. Microscopic quantification of interstitial fibrosis (F) and left ventricular thickness global (G) and in the fibrotic areas (H). (I) Representative images from immunohistochemistry stainings using anti-CD31 antibody to detect microvascularization. (J) Quantification of CD31-positive cells in mice treated with saline or sEVs-THN. The mRNA expression levels of col1a1 (K), col3a1 (L), acta2 (M), tgf (N) and ctgf (O) in myocardial samples isolated from sham mice and animals submitted to permanent coronary ligation followed by saline or sEVs-THN treatment. Graphs show mean ± SD values. Data were analysed using unpaired Student's t-test. * p < 0.05 and ** p < 0.01 vs. saline-treated group.

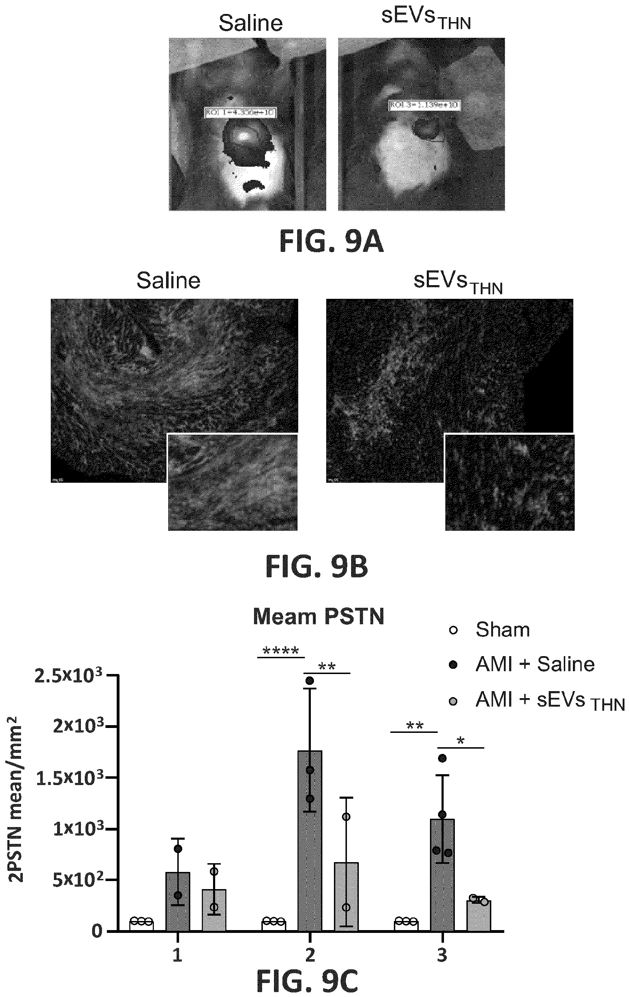

**Figure 9****. Mice strain Pstn/Tmt were analyzed in vivo 28 days after treatment with saline or sEVsTHN shortly after AMI.** (A) in vivo quantification of red fluorescence due to Periostin expression under tomato promoter was performed using the IVIS Spectrum (PerkinElmer; λexcitation: 580; λemission: 610). (B) Criosections of heart tissues showing direct red fluorescence in saline or sEVs-THN and (C) quantification of Periostin signal

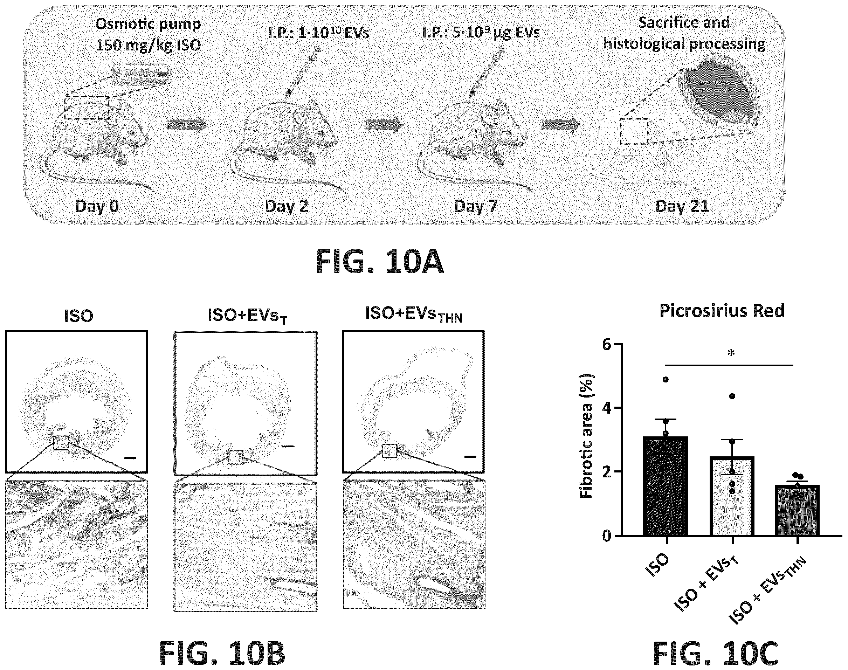

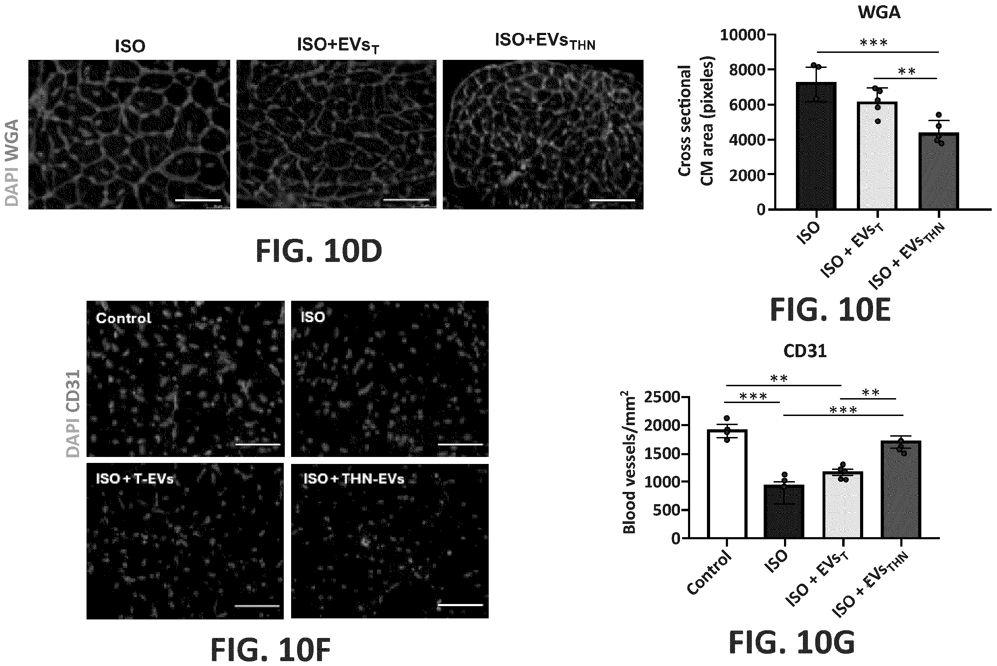

**Figure 10****. sEVs-THN reduce fibrosis and hypertrophy and increase vascular density in a type 2 myocardial infarction model by isoproterenol infusion.** A) Schematic of the isoproterenol cardiac damage model. B) Representative images of picrosirius red staining in mouse hearts subjected to the ISO damage model. Scale bar = 200 µm. C) Quantification of fibrotic area in heart sections. For each animal, the percentage of collagen-covered area relative to the total heart area is plotted. D) Representative images of wheat germ agglutinin (WGA) staining (red) and nuclei staining (DAPI, blue) for each experimental group. Scale bar = 50 µm. E) Measurement of cardiomyocyte cross-sectional area by WGA staining of animal heart slices. Five animals were included in each group. F) Representative images of CD31 staining (red) and nuclei staining (DAPI, blue) to measure microvasculature. Scale bar = 50 µm. G) Quantification of microvasculature using an antibody against CD31. Circular stained structures with an area of up to 23.5 µm² were counted. Five animals were included in each group. ImageJ software was used for image analysis. Data were analysed with ANOVA and Tukey's post hoc test and are represented as mean ± SEM. * p < 0.05, ** p < 0.01 and *** p < 0.001.

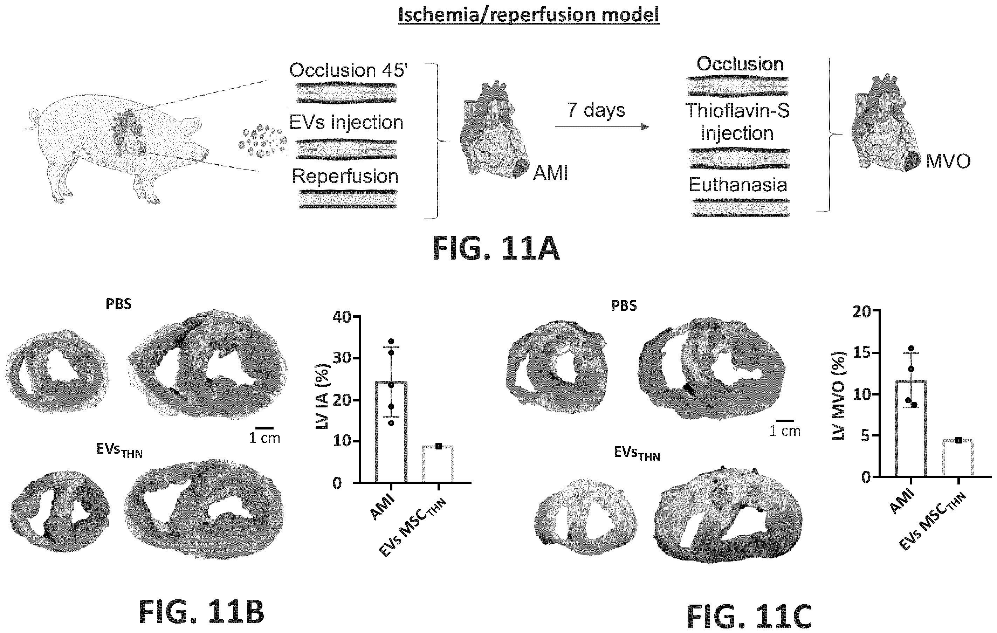

**Figure 11****. sEVs-THN decreases area at risk in a swine in vivo model of I/R.** A) Schematic representation of the pig ischemia reperfusion model. PBS was injected in the control group (I/R). B) Representative images of TTC staining in heart slices and quantification of LV IA (%) by morphometry analysis. C) Representative images of Thioflavine-S staining in heart slices and quantification MVO (%) by morphometry analysis. Animals included in each experimental group: I/R, n = 5; I/R + sEVs-THN, n=1. Data are represented as mean ± SEM..

### Detailed description of the invention

There exists a need for methods and compositions to repair and/or regenerate damaged cardiac tissue due to ischemia, infarction, or other forms of myocardial injury. While promising therapeutic options for the treatment of coronary artery disease have been proposed in the state of the art, the authors of the present invention provide herein EVs compositions, as well as optimized synthetic lipid-based nanoparticles mimicking their functional properties, that yield unexpectedly beneficial effects in the repair and regeneration of damaged cardiac tissue. Specifically, the present invention provides lipid based nanoparticles characterized by molecules uniquely present in EVs derived from genetically modified MSCs compared to native MSCs, as well as those that are inherently present in native MSCs but exhibit markedly increased expression in sEVs-THN.

In a first aspect, the present invention refers to a lipid nanoparticle (LNP), comprising a protein composition that comprises at least one, preferably at least two, of the following proteins: PPP2CB, LTBP4, STX4, INHBA, GNAI2, ENG, and LOXL2; and an miRNA profile comprising at least one, preferably at least two, of the following miRNAs: hsa-miR-191-5p, hsa-miR-342-3p, hsa-miR-139-5p, hsa-miR-127-3p, hsa-miR-222-3p, hsa-miR-495-3p, hsa-miR-26a-5p, hsa-let-7g-5p, and hsa-miR-25-3p. The LNP has a size between 50 and 150 nm, preferably between 90-140 nm.

Regarding the protein profile, the LNPs of the invention are characterized by 7 highly abundant proteins:
PPP2CB protein is found to be enriched in important pathways such as Wnt/β-catenin signaling, Wnt signaling, β-catenin binding, and the Hippo pathway itself together with WNT5A, and SERPINE1, also detected only in sEVsTHN. WIF1, a component of the Wnt pathway, is down-regulated in prostate, breast, lung, and bladder cancer.

The LTBP4 protein (Latent Transforming Growth Factor Beta Binding Protein 4) is known for its role in regulating the bioavailability of TGF-β (Transforming Growth Factor Beta), a cytokine with multiple functions, including development, repair, and pathogenesis in tissues, such as cardiac tissue. LTBP4 binds to TGF-β and keeps it in a latent form until activated, allowing precise regulation of TGF-β signaling during heart regeneration and repair processes. In the context of cardioregeneration, LTBP4 may contribute to controlling cardiac fibrosis, inflammation regulation, and prevention of adverse remodeling by limiting TGF-β bioavailability. At the molecular level, the lack of LTBP4 results in irregular elastic fiber formation and abnormal TGF-β activity. Dysregulated TGF-β signaling has been implicated in various cardiovascular diseases, such as atherosclerosis, cardiac fibrosis, and aortic aneurysm disease. Modulating LTBP4 and TGF-β activity could therefore be a therapeutic strategy to improve cardiac tissue regeneration and function after injury.

Syntaxin 4 (STX4), a target-SNARE protein, plays a critical role in cardiac development by mediating proper vesicular transport and membrane fusion within cardiomyocytes, which are essential for intracellular trafficking and conduction system function. Variants in STX4 are associated with cardiomyopathy, conduction defects, and pleiotropic abnormalities, highlighting its importance in maintaining normal embryonic cardiac morphology and function.

The beta A subunit of inhibin (INHBA) is a protein that is part of both activins and inhibins. It has been found that INHBA acts as a mitogen for cardiomyocytes, promoting their proliferation, and that its overexpression accelerates cardiac recovery and scar resolution after injury.

The alpha subunit of the inhibitory G protein (GNAI2, also known as Gai2) plays a central role in cardiac protection after myocardial infarction. Studies have shown that GNAI2 depletion increases infarct size in mice, while signaling through this pathway protects the heart from ischemic injury.

Endoglin (ENG) is a transmembrane glycoprotein that serves as a co-receptor for transforming growth factor-beta (TGF-β) signaling, playing a crucial role in cardiac muscle development by regulating angiogenesis, vascular integrity, and myocardial remodeling. Specifically, in cardiac muscle development, mutations or dysregulation of endoglin can result in defects in heart development, such as congenital heart disease, impaired vascularization, or abnormal myocardial architecture. Its role highlights the intricate balance between vascular and cardiac muscle development during embryogenesis. Endoglin has a crucial role in blood cell-mediated vascular repair.

Lysyl oxidase-like 2 (LOXL2) is an enzyme that plays a pivotal role in the regulation of angiogenesis, particularly under hypoxic conditions. Exosomes derived from hypoxia-preconditioned dental pulp stem cells (DPSCs) are enriched with LOXL2, which significantly enhances the proliferation, migration, and tube formation capabilities of human umbilical vein endothelial cells (HUVECs). This pro-angiogenic effect is mediated through the transfer of LOXL2 via exosomes, highlighting its crucial function in promoting vascular formation and remodeling in response to low oxygen environments.

In a more preferred embodiment, the protein composition comprises the combination of the 7 proteins PPP2CB, LTBP4, STX4, INHBA, GNAI2, ENG, and LOXL2.

In a particular embodiment, the LNP of the invention further comprises at least one protein relevant by their role in cardicac tissue repair, selected from ITGA1, ITGA3, ITGA4, ITGA6, ITGAV, ITGB5, RHOB, NOTCH2, NOTCH3, ANGPT1, RHOG, MYOF, WNT7A, PDGFRA, IGFBP2, TGFB1, HGF, TIMP1 and NOTCH 1.

Integrins are essential mediators of cell-extracellular matrix (ECM) interactions, playing a crucial role in cardiac tissue's development, homeostasis, and response to injury. Among the integrins associated with cardiac tissue, integrin α5β1 (ITGA5/ITGB1) interacts with fibronectin to regulate tissue remodeling and repair, particularly after myocardial infarction. Laminin-binding integrins such as a7β1 (ITGA7/ITGB1) and α6β1 (ITGA6/ITGB1) are critical for cardiomyocyte differentiation, organization, adhesion, and survival. Collagen-binding integrins, including α1β1 (ITGA1/ITGB1), α2β1 (ITGA2/ITGB1), and α10β1 (ITGA10/ITGB1), are involved in ECM remodeling and maintenance, particularly in the context of cardiac fibrosis. Additionally, integrins αvβ3 (ITGAV/ITGB3) and αvβ5 (ITGAV/ITGB5), which bind vitronectin, play vital roles in angiogenesis, ECM remodeling, and cellular responses to mechanical stress. The proteins MYOF, PDGFRA, IGFBP2, HGF, TGFB1, and TIMP1 play critical roles in regulating key cellular and extracellular processes, particularly in the cardiovascular system. Myoferlin (MYOF) is essential for membrane repair, vesicle trafficking, and endothelial cell function, contributing to vascular integrity and angiogenesis. Platelet-Derived Growth Factor Receptor Alpha (PDGFRA), a receptor tyrosine kinase, plays a vital role in cardiac development, vascular remodeling, and fibrosis. Insulin-Like Growth Factor Binding Protein 2 (IGFBP2) regulates the activity of insulin-like growth factors (IGFs), impacting cell growth, apoptosis, and metabolic processes, and has been implicated in angiogenesis and vascular function. Hepatocyte Growth Factor (HGF), via the MET receptor, promotes cell proliferation, motility, and survival, and enhances vascularization and cardiomyocyte regeneration following cardiac injury, exhibiting significant cardioprotective effects. Transforming Growth Factor Beta 1 (TGFB1) serves as a central regulator of cell growth, differentiation, immune response, and extracellular matrix production, and is a key driver of cardiac remodeling and fibrosis in pathological conditions. Tissue Inhibitor of Metalloproteinases 1 (TIMP1) inhibits matrix metalloproteinases, controlling extracellular matrix turnover, cardiac fibrosis, and ventricular remodeling post-injury. Besides, RHOB, a member of the Rho family of GTPases, contributes to cytoskeletal organization and cellular migration, supporting angiogenesis and tissue remodeling after injury. NOTCH2 and NOTCH3, key components of the Notch signaling pathway, regulate vascular smooth muscle cell differentiation, endothelial function, and repair processes, while NOTCH1 is essential for cardiomyocyte proliferation and angiogenesis during regeneration. ANGPT1 (Angiopoietin-1) promotes vascular stability and endothelial survival, enhancing neovascularization in ischemic tissues. RHOG, another Rho family GTPase, facilitates cell migration and cytoskeletal dynamics, playing a role in tissue repair and angiogenesis. ITGA4 (Integrin α4) mediates cell adhesion and migration, crucial for cardiac repair and inflammatory cell recruitment. WNT7A, through the Wnt signaling pathway, drives cardiomyocyte proliferation and tissue remodeling, contributing to regeneration. Together, these proteins orchestrate cellular and molecular mechanisms that underlie cardiac regeneration, and their presence in EVs-THN could be at the onset of therapeutic effects observed after infusion following myocardial injury.

Furthermore, the nanoparticles of the invention comprise proteins that already exist in the sEVs obtained from the MSC-T, before being modified, but that, by overexpressing HIF1α and N1ICD, increase their concentration in the sEVs.

Therefore, in another particular embodiment, the expression levels of at least one of the following proteins are increased by at least two fold in the LNPs of the invention: ENO1, PXDN, ITGB3, MAMDC2, NID2, HSPG2, KRT9, COL4A2, FBN1, SVEP1, LAMA4, COL3A1, ANGPTL2, LAMA2, COL18A1, LAMB2, ADAM10, FGG, YWHAG, LAMb1, YWHAQ, VWF, HSP90AB1, EMILIN1, PDCD6IP, COL6A2, NID1, FBN2, SRPX, GPC1, LAMC1, FLNA, COL6A1 and POSTN.

Regarding the miRNA cargo, the nanoparticles of the invention are characterized by the following highly expressed miRNAs:
hsa-miR-191-5p: miR-191-5p enhances cell viability and reduces apoptosis in primary human cardiomyocytes subjected to hypoxia, suggesting a cardioprotective role for this miRNA against I/R damage.

hsa-miR-342-3p: This miRNA has been found downregulated in the serum of patients with chronic heart failure. This miRNA is downregulated in plasma exosomes of post-Ml convalescent patients, impacting the cardioprotective potential of these exosomes in a rat permanent LAD ligation model. Conversely, injecting plasma exosomes from healthy controls, rich in this miRNA, attenuates myocardial injury by acting on SOX6-mediated apoptosis and TFEB-mediated autophagy.

hsa-miR-139-5p: Like miR-342-3p, miR-139-5p is also part of the serum miRNA signature found downregulated in chronic heart failure patients. The cardioprotective effect of this miRNA has been investigated in several studies. Overexpression of miR-139-5p counteracts the hypertrophic effects of isoproterenol infusion by inhibiting c-Jun, preventing ANP and BNP expression, and cardiomyocyte hypertrophy. miR-139-5p regulates apoptosis and autophagy via the AMPK/mTOR/ULK1 signaling pathway, protecting cardiomyocytes from I/R damage.

hsa-miR-127-3p: Patients who have suffered a myocardial infarction (MI) have been observed to exhibit reduced serum levels of miR-127-3p. Overexpression of this miRNA in cardiomyocytes leads to reduced apoptosis by targeting its gene, CDKN3.

hsa-miR-222-3p: In cardiac biopsies from patients with severe fibrosis and dilated cardiomyopathy or aortic stenosis, miR-222-3p levels are significantly lower compared to patients with non-severe fibrosis. Furthermore, in patients with aortic stenosis, levels of this miRNA correlated negatively with the extent of cardiac fibrosis and left ventricular stiffness. Additionally, in a pressure overload model in mice induced by angiotensin II infusion, overexpression of miR-222-3p exerted an anti-fibrotic effect by counteracting the TGF-β signaling pathway.

hsa-miR-495-3p: Various studies have examined the role of miR-495-3p in ischemia/reperfusion (I/R) injury and cardiac fibrosis. Suppression of this miRNA (through different pathways) aggravated I/R-induced damage in cardiomyocytes. Conversely, reducing miR-495-3p promoted fibrosis both *in vitro* and *in vivo.*

hsa-miR-26a-5p: miR-26a-5p has been shown to regulate various biological processes in many cell types. In the cardiovascular context, its levels are reduced after infarction. Additionally, injection of this miRNA protects cardiomyocytes from I/R damage. Overexpression of miR-26a-5p reduces collagen I expression in cardiac fibroblasts. Moreover, it also regulates autophagy in these cells, helping to control the fibrosis process.

hsa-let-7g-5p: This miRNA has been associated to the control of chronic inflammation. miRNA fingerprint in a cohort of 53 patients with acute myocardial infarction (AMI) with non-ST-segment elevation (NSTEMI) relative to miRNA expression in healthy controls revealed that levels of et-7g-5p, let-7e-5p, and miR-26a-5p expression was inversely associated with serum levels of pro-inflammatory cytokines TNF-α, and the chemokines MCP-3 and MDC, suggesting the role of this miRNA in the context of ischemic heart diseases. Moreover, the expression of let-7g-5p was significantly downregulated during sympathetic hyperactivity during chronic heart failure.

hsa-miR-25-3p: miR-25-3p transfer, mediated by exosomes derived from mesenchymal stem cells, protects against ischemia/reperfusion injury by reducing M1 macrophage polarization. This suggests that miR-25-3p has an anti-inflammatory and protective effect in the context of cardiac injuries, modulating immune responses and reducing tissue damage. Similarly, the role of miR-25-3p contained in exosomes derived from mesenchymal stem cells in attenuating myocardial infarction. The results suggest that exosomal miR-25-3p mitigates myocardial infarction by targeting pro-apoptotic proteins and EZH2, an epigenetic regulator. This indicates that miR-25-3p has a protective effect in myocardial infarction, modulating apoptosis and cellular responses to cardiac damage.

In a more preferred embodiment, the miRNA profile comprises the combination of the 9 miRNAs: hsa-miR-191-5p, hsa-miR-342-3p, hsa-miR-139-5p, hsa-miR-127-3p, hsa-miR-222-3p, hsa-miR-495-3p, hsa-miR-26a-5p, hsa-let-7g-5p, and hsa-miR-25-3p.

In the present invention, the term "lipidic nanoparticle (LNP)" refers to a colloidal structure at the nanometric scale, preferably ranging in size from 50 nm to 150 nm, predominantly composed of lipids, lipid derivatives, or combinations thereof. These nanoparticles can be of natural origin, derived from biological sources such as plants, animals, microorganisms, or cells, or synthetic, produced through chemical or biotechnological methods using functionalized or artificially modified lipids. The LNPs can adopt various structural configurations, including: Extracellular vesicles (EVs), naturally produced by cells; Lipid-based exosomes, derived from EVs; Lipid vesicles, such as liposomes, characterized by one or more lipid bilayers surrounding an aqueous core; Solid lipid nanoparticles (SLNs), which feature a solid lipid matrix capable of encapsulating bioactive compounds; emulsion-like lipid nanoparticles, containing higher molar percent of the ionizable lipids; Nanostructured lipid carriers (NLCs), which combine solid and liquid lipids to enhance payload capacity and stability; Hybrid formulations that combine lipids with other materials, such as polymers like PEG or stabilizing agents. The lipids constituting these nanoparticles include, but are not limited to: Phospholipids, such as phosphatidylcholine or phosphatidylethanolamine; Triglycerides, both short- and long-chain; Fatty acids and their derivatives; Sterols, such as cholesterol or structural analogues; Ceramides and membrane lipids; Functionalized or polymerized lipids of synthetic origin.

Lipid nanoparticles can be incorporated into systems known as lipid formulations, where they serve as a basis for enhancing the delivery and performance of active compounds. By forming part of these formulations, lipid nanoparticles contribute to improved solubility, stability, bioavailability, and controlled release in pharmaceuticalnapplications. Lipid formulations can include various types of LNPs, such as liposomes, emulsions, SLNs, EVs, etc.

According to the above, the invention encompasses both LNPs generated through natural processes and those manufactured or modified using chemical, biological, or hybrid technologies.

In a particular embodiment, the LNP can be selected from the group comprising:
a) nanoparticle obtained from a natural source, and
b) synthetic nanoparticle obtained from chemical or biological synthesis methods

In a particular embodiment, the LNP is an EV naturally derived from modified parental cells.

Preferably, the parental cells are mesenchymal stem cells from different tissular sources or cardiac progenitor cells, native or iPSC derived. In a particular embodiment, the LNP obtained from a natural source is an EV derived from immortalized dental pulp stem cells.

In a preferred embodiment, the mesenchymal stem cells are genetically modified to overexpress hTERT, HIF1α and N1ICD.

According to the above, in a second aspect, the present invention refers to a method for obtaining EVs isolated from MSCs genetically modified to overexpress HIF1α, hTERT and N1ICD, that comprises the steps of:
- Immortalizing a culture of MSCs by overexpressing the human telomerase enzyme (hTERT) by lentiviral vector, obtaining MSC-T,
- expand the MSCs to a required quantity,
- Overexpressing HIF1α and N1ICD in the immortalized MSCs-T, by lentiviral infection, obtaining MSCs-THN, wherein the overexpression of N1ICD is induced by an inducible promotor, and
- Isolating the sEVs released by the MSCs-THN to the culture medium.

While constitutive expression of HIF1α does not affect MSC proliferation, expression of N1ICD stops the cell cycle and cells stop proliferating, so it is necessary to first expand the MSCs to the amount necessary to obtain the desired amount of EVs, and then induce N1ICD expression using an inducible promoter and start collecting sEVs.

Therefore, the use of an inducible promoter is essential, since overexpression of N1ICD under a constitutive promoter leads to these cells stopping their growth (even when they overexpress hTERT).

In another particular embodiment, the LNP of the invention is a synthetic nanoparticle obtained from chemical or biological synthesis methods, such as emulsion-like LNPs (eLNPs), membrane-rich LNPs (mLNPs) or classic "bleb" structure LNPs (cLNPs). In a preferred embodiment, the synthetic LNP is a synthetic liposome.

Typically, lipid-based architectures for medical applications require an average diameter smaller than 100 nm. The prospective nanoarchitectures can be simple small unilamellar vesicles (SUVs) or more complex LNPs or lipoplexes. The distinction between these is that generally, SUVs (or liposomes) are spherical vesicles with a lipid bilayer encapsulating an aqueous core, while LNPs are solid or semi-solid particles primarily composed of lipid aggregates, often lacking a distinct bilayer structure.

Lipid-based nanocarriers enable targeted delivery and controlled release by surface modification and composition alteration. Active targeting can be achieved by attaching functional ligands, antibodies and carbohydrate moieties to the vesicle surface, which selectively bind to the specific receptors and antigens on the surface of the targeted cell, in this case, proteins/integrins that direct these vesicles to the myocardium. Furthermore, cargo can be loaded passively or actively. Passive encapsulation loads molecules of interest as the self-assembly of vesicles occurs. By contrast, active cargo loading, oftentimes achieved by the pH gradient method, drives cargo into preformed lipid vesicles. Generally, passive loading often has relatively low encapsulation efficiency while active loading can reach extremely high encapsulation efficiency

These nanoparticles are capable of encapsulating or associating active ingredients, biomolecules, nucleic acids, peptides, proteins, or chemical compounds, enabling applications in diverse fields, including controlled drug delivery, gene therapies, diagnostics, cosmetics, and nutraceuticals.

Accordingly, in another aspect, the present invention refers to the use of the LNPs, as a nanocarrier, nanovector and/or nanocapsule for the release and/or encapsulation of molecules.

Due to the special features of the LNPs of the invention, they can be used to formulate pharmaceutical and/or therapeutical compositions.

Thus, in another aspect, the present invention provides a pharmaceutical and/or therapeutical composition comprising a therapeutically effective amount of one or more LNPs as defined above, and optionally together with other appropriate pharmaceutically acceptable excipients and/or carriers.

As it is shown in the examples of the present invention, the LNPs of the invention are effective in the treatment of ischemic heart diseases.

Thus, in another aspect, the present invention provides a LNP as defined above for use as a medicine in the treatment of ischemic heart diseases, in particular MI. This aspect can also be formulated as the LNPs of the invention for use in a method of regenerating cardiac tissue in an individual having damaged cardiac tissue, preferably by ischemia, wherein the method comprises administering one or a more than one of said LNPs to the cardiac tissue of said individual, wherein after administration of said one or more nanoparticles, viability of the damaged tissue is improved or formation of new tissue is facilitated.

### Examples

### Materials and Methods

### 1. Animals

Adult C3H/HeNCrI and C57/BL6, male mice (10-14 weeks old, weighting 35-40 g) were selected for in vivo studies (Charles River Laboratories,Wilmington, MA, USA). The inbred strain (B6;129S6-Gt(ROSA)26Sortm14(CAG-tdTomato)Hze/J) was also used, named Tmt/Pstn obtained after crossing mice B6129S-Pstn and B6;129S6-Gt(ROSA)26Sortm14(CAG-tdTomato)Hze/J (Jackson laboratories). Only Pst+/-Tmt++ animals were used for the detection of myofibroblasts after injury in myocardial tissue.

The study population was composed of large white female domestic pigs weighing 39.1 ± 4.4 kg ("EI Pampo" farm, Valencia, Spain). All procedures were approved by institutional ethical and animal care committees at the Health Institute La Fe Hospital (approved 2024-VSC-PEA-0198 for mice myocardial infarction type I, 2021/VSC/PEA/0214 for the model of isoproterenol-induced heart failure, and CEEA 2022/VSC/PEA/0198 for swine model of ischemia/reperfusion injury).

### 2. Eukaryotic Cell Lines

Human embryonic kidney (HEK 293T) cells were obtained from the American Type Culture Collection (Manassas, VA, USA). Cells were cultured in high-glucose DMEM containing 10% heat-inactivated fetal bovine serum (FBS) and 1% penicillin/streptomycin solution (P/S), all from Gibco (ThermoFisher Scientific). Dental pulp MSCs in passage 1/2 were obtained from the Spanish National Cell Line Bank (BNLC) through the Inbiobank Foundation (San Sebastián, Spain). MSC were immortalized and characterized through lentiviral transduction with telomerase reverse transcriptase (MSC-T) (*Gómez-Ferrer et al (2021).* MSC and MSC-T were cultured in low-glucose DMEM containing 10% heat-inactivated FBS and 1% P/S, all from Gibco. HCF cells were obtained from PromoCell and cultured in Fibroblast Growth Medium-3 BulletKit^{™} (PromoCell, Heidelberg, Germany). H9C2 cells were purchased from ATCC and cultured in DMEM with high glucose, supplemented with 10% FBS for expansion and maintenance. Human umbilical vein endothelial cells (HUVEC) were purchased from ATCC and cultured in Endothelial Cell Growth Medium -2 Bulletkit^{™} (Lonza). All cells were cultured in a Forma Series II Incubator Model 3141 (ThermoFisher Scientific) at 37 °C and 5% CO2.

### 3. MSC-T Lines Generation

The N1ICD plasmid contains a doxycycline-inducible promoter (Tet-on) because its constitutive overexpression was shown in previous experiments to arrest MSC growth. Genetically modified MSC-T cells for N1ICD overexpression contained a Tet-On inducible vector system whose expression depends on the presence of doxycycline (10 µg/mL; Sigma-Aldrich). Lentiviral-transduced MSC-T cells were seeded under the same conditions, but standard FBS was replaced for heat-inactivated tetracycline-free FBS (BioWest, Nuaillé, France) to allow the expression of the vector encoding proteins only when doxycycline was added to the medium. HEK 293T cells were used as packaging cells to produce lentiviral particles, using third-generation lentiviral transduction protocol (Elegheert, J.; et al. Lentiviral Transduction of Mammalian Cells for Fast, Scalable and High-Level Production of Soluble and Membrane Proteins. Nat Protoc 2018, 13, 2991, doi:10.1038/S41596-018-0075-9). After titration, an adequate multiplicity of infection of viral particles was used to transfect MSC-T, and 8 µg/mL polybrene (Sigma-Aldrich) was added to increase infection rate.

### 4. Isolation of EVs

The sEVs recollection medium (RM) contained low-glucose DMEM with 10% tetracycline and vesicles-free FBS and 1% P/S. Doxycycline (Sigma-Aldrich) was added at a final concentration of 10 µg/mL and refreshed every day. Vesicles naturally present in FBS were removed by ultracentrifugation at 150,000× g for 16 h at 4 °C, followed by filtration through a polyether sulfone membrane filter with a pore size of 0.22 µm, as described [*Théry, C.; et al. Isolation and Characterization* of *Exosomes from Cell Culture Supernatants and Biological Fluids. Curr Protoc Cell Biol 2006, 30, 3.22.1-3.22.29, doi:10.1002*/*0471143030.C80322S30*]. Cells were incubated in sEV RM with doxycycline for 48 h before supernatant collection for SEV isolation. Isolations were performed in batches of 250 mL of supernatant.

sEVs were isolated by tangential flow filtration (TFF) followed by size exclusion chromatography (SEC). First, centrifugation is performed at 2,000 g for 20 min at 4°C (Eppendorf 5804 benchtop centrifuge, rotor A-4-62) to remove cell debris. The supernatant is then passed through a 0.22 µm filter and concentrated using TFF-Easy filters (HansaBiomed Life Sciences). The TFF-Easy is a hollow fibre filter cartridge made of polysulphone, which allows the concentration of small molecules and vesicles. The concentration results in a volume reduction of approximately 20 times. Next, SEC is performed to separate the EV fraction from other molecules such as proteins. This process is carried out using commercial 2 ml EVs sEC Columns (StemCell Technologies).

### 5. Transmission Electron Microscopy

Isolated sEVs loaded onto pure carbon-coated copper grids, fixed with 2% PFA and 1% glutaraldehyde, contrasted with 1% uranyl acetate, and embedded in 0.4% methyl cellulose for analysis by negative staining. The grids were examined with a FEI Tecnai G2 Spirit transmission electron microscope (Thermo Fisher Scientific). All images were acquired using a Morada digital camera (Olympus Soft Image Solutions GmbH, Münster, Germany).

### 6. Nanoparticle Tracking Analysis

NTA was used to determine the sEVs concentration and particle size distribution. All particle tracking analyses were performed using a LM14C Nano Sight instrument (Malvern Instruments Ltd., Malvern, UK), using the same settings (camera level 16, 3 videos of 90 s and 1300/512 slider shutter/gain, respectively), at the proper concentration, to obtain around 50 particles/frame. An analysis of the acquired videos was performed with threshold 5 and gain 12 with the proprietary NTA v3.2 software.

### 7. Single-Particle Fluorescence and Interferometry Imaging with ExoView^{®}

ExoView^{®} platform is based on single-particle interferometric reflectance imaging detection of sEVs captured on a silicon substrate chip, constituted by an array of spots printed with different antibodies. This can be combined with sEVs immunostaining with fluorophore-conjugated antibodies, followed by fluorescence imaging, and can be multiplexed for the simultaneous detection of up to three distinct fluorophores. Isolated sEVs were diluted 1:10 in incubation solution (1×); 50 µL of this sample was carefully pipetted onto the silicon chip coated with individual antibody spots against human CD9, CD63, and CD81, as well as negative isotype controls. After overnight incubation in a 24-well plate, chips were washed three times on an orbital shaker orbital at 500 rpm for 3 min with Solution A. Then the chips were incubated as before at 500 rpm for 1 h at room temperature with a cocktail of fluorescent antibodies (anti-CD81-AF488, anti-CD9-AF555, and anti-JAG1-AF647) diluted in Blocking Solution. Chips were washed once in Solution A, three times in Solution B, and once in deionized water. Chips were carefully removed from the 24-well plate, washed further in deionized water, and removed for drying. Image and data acquisition for each chip was performed with the ExoView^{®} R100 (NanoView Biosciences), and a data analysis was performed with ExoView Analyzer 3.1.4. An antibody to JAG1 was purchased from R&Dsystems (FAB1277R-100UG).

### 8. HCF Stimulation In Vitro

HCF were seeded at 2,5 ×10⁴ cells/cm² and were allowed to attach for 24 h. Thereafter, culture medium was replaced with starvation medium (high-glucose DMEM, 0.5% vesicle-depleted FBS, 1% non-essential amino acids, and 1% P/S). After 16 h, fibroblasts were stimulated with 100 µM L-ascorbic acid 2-phosphate (Sigma-Aldrich), 3.16 ng/mL recombinant TGFβ-1 (R&D Systems, Minneapolis, MI, USA), and 3.16 µg/mL dextran sulphate (Sigma-Aldrich), as described [Palano, G.; et al. A High-Content, in Vitro Cardiac Fibrosis Assay for High-Throughput, Phenotypic Identification of Compounds with Anti-Fibrotic Activity. J Mol Cell Cardiol 2020, 142, 105-117, doi:10.1016/J.YJMCC.2020.04.002*.*]*.* Treatments were added at the same time as the media exchange. Unstimulated cells (cells starved but not stimulated) and untreated cells (neither starved nor stimulated, control) were used as control conditions. HCF were stimulated for 48 h before proceeding to readouts.

### 9. Migration Assay

HCF cells were seeded in 48-well plates with 4×10⁴ cells per well, with the aim that the next day each well would be at a suitable confluence for the assay (around 80%). Stimulation medium and treatments were then added and incubated for a further 24 hours. After this time, the cell monolayer in each well was evenly incised using a pipette tip to create a 'wound' or cell-free area. The wells were then carefully washed to remove any remaining loose cell debris and the culture medium was changed to GMF-3. Then, initial (time 0) images of the wounds were acquired, and then at different times until the wound was closed in one of the conditions (in our case 24 hours). Finally, Imaged image analysis software (version del software) was used to quantify the area closed in each image and calculate the percentage of wound closure as a function of time.

### 10. Matrigel Tube Formation Assay

First, plates were prepared with 50 µl of Matrigel Growth Factor Reduced (BD Biosciences) in each well of a 96-well plate. The plate was centrifuged for 12 seconds at low speed to evenly distribute the Matrigel on the bottom of each well, and then incubated for 15 minutes at 37°C for the Matrigel to solubilize. Once the plate was prepared, 5×10³ HUVEC cells were seeded per well and the appropriate treatments were added and incubated under hypoxic conditions for 9 hours. After this, images were acquired on a Leica DM6000 inverted microscope, and different parameters related to angiogenesis were analyzed with Wimasis WimTube software (version software).

### 11. CCK-8 Assay

H9C2 cells were seeded in a 96-well plate at a density of 1×10⁴ cells/well. The next day, the medium was changed to deoxygenated DMEM medium without glucose, glutamine and phenol red (Gibco) and placed in an incubator under hypoxic conditions (≤ 1% O2). These oxygen and glucose deprivation (OGD) conditions were used to simulate the stress suffered by cardiomyocytes in the context of coronary occlusion. The cells remained in OGD conditions with or without sEVs for 7 hours. After this time, a 10% solution of CCK-8 reagent (casa commercial) was added to the cells in the culture plate and incubated for a further 1-2 hours under normoxic conditions. Finally, the absorbance of the formazan solution was measured at 460 nm using a plate reader. The absorbance intensity is proportional to the number of viable cells present in the culture.

### 12. Quantification of LDH Activity

In order to measure the cytotoxicity induced by the OGD model in H9C2 cells, the enzyme lactate dehydrogenase (LDH) was quantified using the commercial Cytotoxicity Detection Kit LDH (Roche), following the manufacturer's instructions. For this purpose, H9C2 cells were seeded in 96-well plates at a density of 1×10⁴cells/well. The next day, the cells were incubated in the previously described OGD conditions with or without sEVs for 7h. After this time, the supernatant was transferred to a new 96-well plate and the kit reagents were added according to the manufacturer's instructions. The reaction between the reagent and the LDH present in the culture medium produces a colour change that can be measured at 492 nm using a multiwell plate reader. The higher the absorbance, the greater the amount of LDH released and thus the greater the cytotoxicity.

### 13. DCFH-DA

To measure the level of ROS generated by the OGD model, 2×10⁴ H9C2 cells/well were seeded in 24-well plates. The next day, the cells were incubated in the previously described OGD conditions with or without sEVs for 7h. After this time, DCFH-DA (Sigma-Aldrich) was added to the cell culture medium at the concentration recommended by the manufacturer and incubated for 20 min. For cells in normoxia, the wells were washed and incubated with the reagent in medium without FBS. DCFH-DA is a molecule that can penetrate cells and is converted to dichlorofluorescein (DCFH) when taken up by cells. Inside cells, DCFH is converted to oxidised DCFH in the presence of ROS. The greater the amount of ROS in the cells, the greater the amount of DCFH that will be oxidised. The oxidized DCFH emits green fluorescence and allows quantification of the amount of intracellular ROS by flow cytometry. After 20 min of incubation, the cells were lifted with trypsin and visualised with a BD FACSCanto^{™} II cytometer (BD Biosciencies). Cytometry results were analysed with FlowJo software (FlowJo LLC Indicar versión).

### 14. MitoSOX

MitoSOX^{™} (Invitrogen) is a fluorogenic compound used to measure superoxide production in mitochondria. Superoxides are a form of ROS that are generated in mitochondria and can have important effects on cell function and oxidative stress. To assess the level of mitochondrial superoxide in H9C2 cells subjected to OGD, cells were seeded at a density of 20,000 cells/well in 24-well plates. The next day, the cells were subjected to the previously described OGD conditions with or without sEVs for 7 hours. After this time, MitoSOX was added to the medium at the concentration recommended by the manufacturer (500 nM) and incubated for 30 minutes. In the case of normoxic cells, the wells were washed and incubated with the reagent in medium without FBS. After 30 min of incubation, the cells were lifted with trypsin and visualised with a BD FACSCanto^{™} II cytometer (BD Biosciencies). Cytometry results were analysed with FlowJo software (FlowJo LLC).

### 15. Inmunofluorescence

HCF cells were fixed with 4% PFA for 10 min at 37 °C. After washing with PBS, cells were permeabilized with 0.2% Triton in PBS for 1 h at room temperature and blocked for nonspecific epitopes with 2% BSA in PBS (both from Sigma-Aldrich). Thereafter, samples were incubated with primary antibodies solution, containing 0.1% BSA in PBS. Mouse anti-αSMA (dilution 1/100; clone 1A4; Dako) was used. Next, samples were washed 3 times with PBS and incubated with an α-mouse Alexa Fluor 555 secondary antibody (Life Technologies) and DAPI for 1 hour at room temperature. Images were taken using CellVoyager CV8000 high-throughput screening system (Yokogawa, Tokyo, Japan), and fluorescent signals were analyzed with Leica Application Suite Version 2.4.0 R1 version and imaged 1.53t software (NIH).

### 16. Proteomic Analysis of sEVs

To analyse the protein content of the sEVs, proteomic analysis of the different types of sEVs (sEVsT, and sEVsTHN) was performed, as previously described [79]. The proteomic analysis was performed at the proteomics facility of the SCSIE University of Valencia, belonging to Proteored, PRB3, and is supported by grant PT17/0019, from the PE I+D+i 2013-2016, funded by ISCIII and FEDER.

Proteins were quantified with Qubit protein BR Assay kit (Thermo Fisher Scientific) following the manufacturer's instructions. 20 µg of protein samples were used for digestion and after, 20 µl of the sample was finally mixed with 1 µL of peptide mixtures in an Evotip pure tip (EvoSep) according to manufacturer instructions. Tandem mass spectrometry analysis, LC-MS/MS, was performed in a Tims TOF fleX mass spectrometer (Bruker). The PASER system (Bruker) was used to search the MS and MSMS data with the Sequest algorithm (ProLuCID) with the following parameters: SwissProt 23.03.10 database; Trypsin specificity; 2-iodoacetamide cys-alkylation and taxonomy not restricted. All identified proteins have an FDR ≤1% and were ordered in function of the Normalized spectral abundance factor (NSAF) value.

Bioinformatics analyses were performed using STRING database (https://string-db.org/) to identify Gene Ontology biological processes. The results were represented graphically using the ggplot2 package (Wickham 2011) from R (R Development Core Team 2008). Venny diagrams were obtained using Venny 2.1.0 (Oliveros, J.C. (2007-2015) Venny. An interactive tool for comparing lists with Venn's diagrams. https://bioinfogp.cnb.csic.es/tools/venny/index.html).

### 17. miRNAseq of sEVs

sEVs were resuspended in PBS and sent to the Genomics Unit of the Centre for Genomic Regulation in Barcelona, where sequencing of miRNAs from sEVs was performed. Libraries were prepared using the NEBNext^{®} Small RNA Library Prep Set from Illumina^{®} (E7330), according to the manufacturer's instructions. Briefly, 1-5 ng of RNA isolated from the sEVs were subjected to 3' and 5' adapter ligation and first-strand cDNA synthesis. Amplification of the libraries was performed by PCR using the indexed primers provided in the kit. All purification steps were performed with AgenCourt AMPure XP beads (ref. A63882). The final libraries were analysed with the Agilent Bioanalyser (5067-4626) to estimate the quantity and check the size distribution. A pool was run for size selection using Novex TBE PAGE 6% gels (EC6265BOX) and the final set was quantified by qPCR using the KAPA Library Quantification kit (KK4835, KapaBiosystems). Finally, they were sequenced on the Illumina NextSeq2000.

Sequencing analysis was carried out by the Multivariate Statistical Engineering Group (GEIM) of the Polytechnic University of Valencia. The FASTQ files were treated with Cutadapt and aligned with Kallisto to obtain the table of counts of each miRNA. Several differential expression methods were used to search for altered miRNAs between groups: edgeR negative binomial test, lime regression models and NOISeq non-parametric test. The edgeR model was applied to the non-normalised miRNA counts and the normalisation provided by CQN, while the limma model was applied only to the CQN-normalised data, which included a logarithmic transformation of expression values. CQN-normalised data without log-transformation were analysed with the NOISeq model. A cut-off p-value of 0.05 was used to select differentially expressed miRNAs from edgeR and limma results, while a cut-off value of 0.10 was used for the FDR-adjusted p-value of NOISeq. Those miRNAs with cumulative counts above 100 were selected and functional enrichment analysis was performed on the selected miRNAs through the GeneCodis platform for both the Gene Ontology and KEGG databases.

### 18. Mouse model of permanent coronary artery ligation

Type 1 myocardial infarction was induced in mice (n=16) by ligation of the left anterior descending coronary artery. As per protocol, the knot was tightened to occlude the coronary artery. Once ischemia onset was confirmed both visually and by electrocardiogram, the mice thorax was closed. After coronary occlusion, animals were divided into two groups depending on the treatment: intracardiac saline (n=8) or 2·1010 sEVsTHN (n=8). A sham group (n= 5) was also included in the study, which underwent the same surgical protocol except for coronary artery occlusion. Intraperitoneal buprenorphine (0.05 mg/kg, twice daily) and meloxicam (0.3 mg/kg, once daily) was administrated for five days after surgery.

### 19. Isoproterenol In Vivo Model

All experimental procedures involving the use of animals were approved by the institutional ethical and animal care committees according to guidelines from Directive 2010/63/EU of the European Parliament on the protection of animals used for scientific purposes, enforced in Spanish law under Real Decreto 1201/2005. Regional Institutional Animal Care and Use Committee authorized the mouse model of isoproterenol-induced heart failure (procedure 2021/VSC/PEA/0214, Generalitat Valenciana, Valencia, Spain). Adult C3H/HeNCrl male mice (10-14 weeks old, weighting 35-40 g) were selected for in vivo studies (Charles River Laboratories, Wilmington, MA, USA). Mice were randomly distributed into three experimental groups (n = 5 in each group): control (ISO), mice treated with T-sEVs (ISO + sEVsT), and mice treated with sEVsTHN (ISO + sEVsTHN). Isoproterenol (I5627, Sigma Aldrich, St. Louis, MO, USA) was infused via osmotic pumps implanted subcutaneously, using a dose of 150 mg/kg/day). Intraperitoneal injection of 2·1010 sEVsTHN was performed just after the first isoproterenol injection. One week later, mice received a second intraperitoneal sEVsTHN injection (1-1010 particles). Equivalent volumes of PBS were injected in the ISO group. Euthanasia was conducted after 21 days, and cardiac tissues were resected for further analysis.

### 20. Cardiac magnetic resonance

Cardiac magnetic resonance was performed on a fully integrated system (MR Solutions, Guildford, UK) consisting of a Cryogen-Free, Dry Magnet 3.0-T MRI scanner (MRS*DRYMAG 3017- 3.0T). During the entire protocol, animals were anesthetized under isoflurane anaesthesia (2% in 100% oxygen; Abbott Laboratories, Chicago, IL). In 0.2 mL of saline, 0.1 mmol/kg of gadolinium-diethylenetriaminepentaacetic acid (Magnograf, Juste S.A.Q.F., Madrid, Spain). Afterwards, mice were placed in a supine position on a pre-heated (37° C) stage.

A low-resolution gradient echo scout scan with zero offset was used to determine the position of the heart in the scanner in coronal, axial, and sagittal directions and afterwards cine images were acquired. A multi-slice short-axis flash sequence (field of view 40 × 40 mm2, matrix 256, slice thickness 1 mm, pulse repetition time/echo time 16/0 msec, flip angle 25°) was used to acquire 4-5 slices in a mid-left ventricular position, perpendicular to the long axis of the heart. The same sequence was used to acquire 2- and 3-chamber slices. Six temporal frames were acquired per cardiac cycle. Late gadolinium enhancement imaging was performed in the same projections used for the cine images, 45 min after contrast injection, employing a multi-slice short-axis flash sequence (field of view 40 × 40 mm2, matrix 256, slice thickness 1 mm, pulse repetition time/echo time 16/0 msec, flip angle 25°). The Retrospective triggering graphical user interface software (Gustav Strijkers, Amsterdam UMC, version 9.1.1) was used for retrospectively gated scanning.

### 21. Histology

Mice hearts were collected at day 21 after isoproterenol treatment, fixed in 2% PFA, embedded with paraffin, and sectioned into 5 µm slices for histological analysis by Picrosirius red and WGA staining. One part of the tissue sections was stained for 30 min in a solution of 0.1% Sirius red in saturated aqueous picric acid for collagen bundle staining, as described [80]. Samples were prepared and images captured with a Leica DMD108 microscope. ImageJ was used for whole-collagen-area quantification. A second batch of tissue sections were stained with 1 µg/mL WGA conjugated to Texas Red-X (Invitrogen, Thermo Fisher Scientific, Waltham, MA, USA). To visualize cell nuclei, DAPI staining was used, and the slides were mounted with FluorSave^{™} Reagent (Merck Millipore, Burlington, MA, USA). Images were captured with a Leica DM2500 fluorescence microscope and analyzed by imaged for the cross-sectional area of cardiomyocytes. Vessel density of cardiac tissue was assessed by staining tissue sections with an α-CD31 antibody (dilution, 1/200; sc-1506; clone ID, M-20; Santa Cruz Biotechnology, Dallas, TX, USA). Heat-induced antigen retrieval (HIER) was conducted prior to antibody labeling. Images were captured with a Leica DM2500 fluorescence microscope and analyzed in ImageJ to measure the number of vessels/mm². Images were captured with a Leica DM2500 fluorescence microscope and analyzed in imaged to measure the perivascular area covered with POSTN.

### 22. In Vivo Swine Model of Ischemia-Reperfusion

Animals were randomized into two groups: I/R (n=5) and I/R + sEVsTHN (n=1). Doses were extrapolated from the in vivo mouse model: 2,4×10¹⁰ µg kg⁻¹ dissolved in PBS. PBS alone was infused in the I/R group. The antiplatelet aggregation drug Clopidogrel (125 mg, Chiesi Inc., USA) was administrated 24 h before the surgical procedure to prevent platelet aggregation during surgery. Animals were preanesthetized with intramuscular ketamine (Ketolar, 15 mL, 50 mg mL⁻¹, Pfizer, USA) before tracheal intubation. To induce anesthesia, 200 mg of the sedative propofol (Sandoz, Holzkirchen, Germany) was intravenously administrated. Additional medication in physiological saline was administrated at 150 mL h⁻¹: 150 mg amiodarone (Trangorex, antiarrhythmic agent, Sanofi, Barcelona, Spain); 10 mg metasedin (analgesic, Esteve, Barcelona, Spain); 15 mg midazolam (sedative, Hameln Pharma Ltd, Gloucester, UK). 1 g of amoxicillin (antibiotic, Sundent Pharmaceutical Co., Ltd., Shanghai, China) was intravenously injected to prevent infection. Animals were maintained in a surgical plane of anesthesia with inhaled sevoflurane (2-3%) through a volume-controlled ventilator (tidal volume 10-15 mL kg⁻¹ and ventilation rate 8-15 breaths min⁻¹). Tidal volume, respiration rate, and aspirated oxygen percentage (FiO2) were adjusted to maintain normocapnia (end-tidal carbon dioxide [EtCO2] and arterial carbon dioxide [PaCO2] 35-45 mm Hg) as measured by a capnometer (NPB-75, Nellcor Puritan Bennett, Boulder, CO, USA) placed on the intubation tube. Electrocardiography (ECG), EtCO2, temperatures (LV, rec rectal,esophageal), and blood pressures (aortic, central venous) were monitored and recorded using a multichannel data acquisition system (AD Instruments, Colorado Springs, CO, USA). One bolus of 1% heparin (5000 UI per 5 mL, Sagent Pharmaceuticals, USA) was administrated at the onset of instrumentation. Occlusion ischemia was induced using a coronary balloon catheter (LVD Biotech Medical Devices, Barcelona, Spain) introduced into the femoral artery and advanced to a mid-left anterior descending artery location typically proximal to the first diagonal branch and inflated to 3-6 atmospheres for 45 min. Vessel occlusion and ischemia were confirmed with contrast dye injection and ST elevation on ECG. A second bolus of 0.5% heparin (2500 Ul per 2.5 mL) was injected 1 h after the first administration. PBS and sEVs_{THN} were infused 5 min before balloon deflation (reperfusion). Each compound was diluted to a final volume of 4 mL at the proper concentration, Loaded in a syringe, and infused through LVD Biotech Medical Devices balloon catheter, which allowed intracoronary administration. One week after the surgical procedure, pigs were anesthetized to perform cardiac magnetic resonance and euthanized by injection of pentobarbital and potassium chloride with confirmation of asystole. Then, hearts were removed surgically by thoracotomy, rinsed twice with physiological saline buffer, and cut into 1.5 cm slices. Blood samples were collected at baseline, after reperfusion, and seven days after AMI to measure cardiac troponin T serum levels by enzyme linked immunoabsorbant assay (ELISA) following manufacturers' instructions (Life Diagnostics Inc., West Chester, PA, USA). The animals were allowed to recover. After 7 days, the procedure was repeated and 20 mL of 4% thioflavin-S (T-S) solution was selectively infused into the LAD using a 2.8 F microcatheter (Progreat, Terumo Japan). Hearts were then arrested with potassiumchloride and excised.

### 23. Triphenyl Tetrazolium Chloride Staining

The LV and RV were sectioned into 5-mm thick short-axis slices. In order to detect T-S stained areas, each slice was viewed from the apical side under ultraviolet light and photographed. Slices were stained by incubation for 20 min in 1% TTC in isotonic pH 7.4 PB at 37 °C for 20 min in the absence of light. Heart slices were then imaged, and regions of infarcted tissue were traced using imaged software (NIH). In the case of rat hearts, 10 slices of 2 mm from apex to atria were used per animal to calculate AAR. In the case of the swine model, the heart was cut in four slices of 1.5 cm. AAR was calculated by measuring the infarcted volume in the four slices and calculated as the volume of infarct in relation to the total heart volume. To calculate the volume of each slice, infarcted and total area were measured on the surface of both slice sides and the volume was estimated assuming a geometry of a truncated pyramid (V = h/3(A + A' + √A × A')).

### 24. Statistics

Three biological replicates (n = 3) were included for all data of in vitro studies. Five animals were included in each experimental group for the in vivo isoproterenol model. Data from all experimental groups were compared using ANOVA, followed by Tukey's post hoc test. All results are presented as mean ± SD. Asterisks indicate statistically significant different between experimental conditions, where p < 0.05 is indicated by *, p < 0.01 by **, and p < 0.001 by ***. Differences were considered at p < 0.05 with a 95% confidence interval. Statistical analysis was performed using GraphPad Prism 8 software (La Jolla, CA, USA).

### RESULTS

### 1. Generation of MSC-HIF1α, MSC-N1ICD and MSC- HIF1α-N1ICD cell lines

For the generation of the N1ICD and HIF1α-overexpressing lines, a hTERT-immortalized MSC line (MSC_{T}) was transduced with the corresponding lentiviral vectors to generate the following lines: MSC_{TH} overexpressing HIF1α, MSC_{TN} overexpressing N1ICD and MSC_{THN} overexpressing both (Figure 1A). Quantification of the percentage of MSC expressing the transgenes was assessed by flow cytometry detecting the GFP fusion protein in MSC_{TH} and MSC_{THN} (Figure 1B) or, in the case of N1ICD, by evaluation of puromycin resistance of transfected parental cells MSC_{TN} and MSC_{THN} (Figure 1C). HIF1α and N1ICD overexpression was assessed measuring mRNA levels (Figure 1D and 1G) and protein expression was confirmed by western blot (Figure 1E, 1F, 1H and 1I). As expected, HIF1α expression was increased in MSC_{TH} while N1ICD expression was increased in MSC_{TN}, and a synergistic action between HIF1α and N1ICD overexpression was observed in MSC_{THN} at both mRNA and protein level.

We next wanted to explore the functionality of both transcription factors in stable modified cell lines. HIF1α overexpression increased hypoxia-activated genes such as the pluripotency gene SRY-Box Transcription Factor 2 (SOX2), which plays a crucial role in maintaining the stem-like characteristics of MSCs, the C-X-C Chemokine Receptor Type 4 (CXCR4), a key chemokine receptor in the mobilization and migration of MSCs to the site of cardiac injury, the Erythropoietin (EPO), a glycoprotein that mediates MSC-cell survival and enhance their anti-fibrotic efficacy and the vascular endothelial growth factor (VEGF) which mediates the differentiation of endothelial progenitor cells into mature cells (Figure 1J). On the other hand, overexpression of N1ICD upregulated the downstream genes Hairy/Enhancer-of-split related with YRPW motif proteins 1 and 2 (HEY1 and HEY2), the Nuclear Factor Erythroid 2-Related Factor 2 (NRF2), together with the primary ligand of the Notch pathway Jagged Canonical Notch Ligand 1 (JAG1) (Figure 1K) indicating that transduction with N1ICD lentiviral vectors resulted into the expression of a functional transcription factor. Thus, overexpression of HIF1α and N1ICD in MSCT increased the expression of genes related to cardiac repair mechanisms, suggesting an enhanced response of MSCTHN and their sEVs in the context of cardiac pathophysiology.

### 2. Characterization of sEVs derived from the different MSC lines

sEVs secreted by MSC-T, MSC-TH, MSC-TN and MSC-THN were harvested from cell culture media and isolated by TFF followed by SEC (see methods) and the resulting sEVs extracts were characterized. The sEVs morphology was analyzed by electron microscopy, with all samples showing a convex morphology characteristic of lipid vesicles (Figure 2A). NTA revealed a similar size distribution in all groups, with most particles having a size between 90 and 150 nm (Figure 2B). sEVs were further characterized using the Exoview platform. No differences in size, or particle concentration were observed among vesicles released by the different cell lines analyzed, indicating that that genetic modifications did not alter the morphology, size and quantity of sEVs (Figure 2C). Immuno-phenotyping of sEVs was performed with antibodies against the most abundant tetraspanins such as CD63, CD9 and CD81. This methodology also allowed the detection of JAG1 in sEVs surface. As the expression of JAG1 in MSC_{TN} and MSC_{THN} was more than 50-fold higher than in MSC_{T} (Figure 2D), it was raised if this protein could also be overexpressed in the surface of sEVs derived from these cells.

When capturing sEVs with chips coated with CD63 antibodies, que observed that the number of sEVs coexpressing CD63/CD81/CD9 was decreased in MSC_{THN} (figure 2D). Interestingly, the presence of JAG1 was detected on the surface of part of the sEVsTN and sEVsTHN (Figure 2D and E), indicating that the shuttle of this antigen in sEVs is promoted by HIF1α and N1ICD overexpression, in line with our previously described results (Gonzalez-King, H.; et al. Hypoxia Inducible Factor-1 α Potentiates Jagged 1-Mediated Angiogenesis by Mesenchymal Stem Cell-Derived Exosomes. Stem Cells 2017, 35, 1747-1759, doi:10.1002/stem.2618*;*). Although sEVsTH also had a slightly higher percentage of JAG1-positive vesicles than the sEVsT, the expression in sEVsTN and sEVsTHN was more evident, suggesting that the main cause of this enrichment in sEVs was the overexpression of N1ICD in parental cells. The molecular profile and functional groups of the sEVsTHN were evaluated by attenuated total reflectance - Fourier transform infrared (ATR-FTIR), revealing no significant alterations with respect to the previously established sEVs profile (Figure F). Protein contributions to the spectrum of sEVs can be observed between 1,200 cm-1 and 1,700 cm-1 and vibrations in the higher region of the spectrum corresponding to the lipid components of the vesicles.

After characterizing the sEVs, it was shought to verify that the factors that were overexpressed in the cells were not inside the sEVs. These factors were overexpressed to make the MSC have suitable characteristics to be used as a source of sEVs: TERT immortalizes them, HIF simulates that they are in hypoxic conditions and NICD enhances the regenerative properties of the MSCs in the biology of the heart. However, it is preferable that these factors do not go inside electric vehicles. Using WB, it was verified that the sEVsTHN did not carry TERT, HIF1α or NICD. Furthermore, it was sought to verify that these EVs carried proteins related to the therapeutic properties they exert and it was found that proteins such as VEGF, creates new blood vessels after injury and collateral circulation to bypass blocked vessels; α-SMA, is a protein prominently found in myofibroblasts and plays a central role in tissue healing and contributes to cell contraction; and SOD-1, protects against damage mediated by free radicals deriving from oxygen. CD81 was used as a control for EV loading (Figure 3).

To further explore the protein content of the sEVs, a proteomics analysis of the sEVs cargo by tandem mass spectrometry, LC-MS/MS, was performed. A total of 654 proteins were exclusively identified in sEVsTHN compared to sEVsT (Figure 4A), suggesting that their expression can be modulated by HIF1α and N1ICD overexpression in parental cells.

Next, a bioinformatic analysis was performed to identify Gene Ontology (GO) biological processes (BP) in which these proteins were involved. GO identified exclusively in sEVsTHN were related to "circulatory system process", "cellular developmental process" and "vesicle-mediated transport" superclusters. Among them, BP related to heart tissue repair and regeneration were selected (Figure 4B). 228 out of 654 proteins of the total list were included in these selected BP. It was proposed that these proteins may contribute to the therapeutic effect of sEVs_{THN}.

In the context of a proteomic assay, NASF often stands for Normalized Spectral Abundance Factor. This is a quantitative measure used to estimate the relative abundance of proteins identified in a mass spectrometry-based proteomics experiment. The general formula is: NASF=Spectral Count/Protein Length. This value can then be further normalized across all proteins in a sample to ensure the sum of NASF values equals 1 or 100%, depending on the scale used.This metric is widely used in shotgun proteomics to compare protein abundances in different samples.

Those 228 proteins exclusively expressed in EVs-THN are showed in table 1.

**Table 1. Proteins exclusively expressed in EVs-THN**

| **Protein** | **NSAF** | **Protein** | **NSAF** | **Protein** | **NSAF** | **Protein** | **NSAF** | **Protein** | **NSAF** |
|---|---|---|---|---|---|---|---|---|---|
| PPP2C B | 2,38342 | LPA | 0,00039 | MAPK3 | 0,00020 | ACTN1 | 0,00011 | SERPINE 1 | 0,00005 |
| LTBP4 | 2,28144 | ENPP2 | 0,00039 | PDGFR B | 0,00020 | COL11A1 | 0,00011 | RAB14 | 0,00005 |
| STX4 | 0,98221 | FGA | 0,00038 | ACTN4 | 0,00020 | ITGA4 | 0,00011 | RFTN1 | 0,00005 |
| INHBA | 0,97899 | IQGAP3 | 0,00037 | ILK | 0,00020 | NRP2 | 0,00011 | MMP14 | 0,00004 |
| GNAI2 | 0,63391 | ANO6 | 0,00036 | PTK7 | 0,00020 | COL2A1 | 0,00011 | TGFB1 | 0,00004 |
| ENG | 0,45637 | DPYSL2 | 0,00036 | NQO1 | 0,00020 | SLC1A5 | 0,00010 | HBB | 0,00004 |
| LOXL2 | 0,34798 | WDR1 | 0,00035 | LPAR1 | 0,00020 | JUP | 0,00010 | PEF1 | 0,00004 |
| BST1 | 0,00330 | ANPEP | 0,00035 | CCBE1 | 0,00019 | CD151 | 0,00010 | HGF | 0,00004 |
| ITGA1 | 0,00281 | LAMA3 | 0,00035 | DDR2 | 0,00019 | EFEMP2 | 0,00009 | NCAM1 | 0,00004 |
| ECM1 | 0,00259 | VASP | 0,00034 | HLA-G | 0,00019 | RAB3D | 0,00009 | HRAS | 0,00004 |
| RTN4 | 0,00214 | TIMP3 | 0,00034 | SLC2A3 | 0,00018 | ZYX | 0,00009 | MEGF10 | 0,00004 |
| ACTA2 | 0,00214 | NPTN | 0,00033 | GNB2 | 0,00018 | FERMT2 | 0,00009 | ATP5F1B | 0,00004 |
| C5 | 0,00171 | ANGPT1 | 0,00032 | WNT5A | 0,00018 | SLC7A1 | 0,00009 | PARK7 | 0,00004 |
| LOX | 0,00164 | SOD3 | 0,00031 | NRAS | 0,00018 | F5 | 0,00009 | PPIB | 0,00003 |
| TPP1 | 0,00154 | KIF5B | 0,00031 | GNA11 | 0,00018 | ATP1B3 | 0,00009 | G6PD | 0,00003 |
| DNAJA2 | 0,00108 | CLIC4 | 0,00031 | BSG | 0,00017 | B4GAT1 | 0,00009 | THBS4 | 0,00003 |
| MST1 | 0,00107 | ALCAM | 0,00031 | FLT1 | 0,00017 | C2 | 0,00009 | COL5A1 | 0,00003 |
| RRAS | 0,00101 | CRMP1 | 0,00031 | RAB13 | 0,00017 | SEMA7A | 0,00008 | C1QC | 0,00003 |
| ACTG2 | 0,00093 | NIBAN2 | 0,00031 | ARHGDI A | 0,00017 | APOE | 0,00008 | RACK1 | 0,00003 |
| ITGA3 | 0,00092 | JAG1 | 0,00030 | CSRP1 | 0,00017 | MYOF | 0,00008 | LTBP3 | 0,00003 |
| ANXA1 | 0,00090 | CD81 | 0,00027 | RALA | 0,00017 | LAMA5 | 0,00008 | PLCB3 | 0,00003 |
| ITGB5 | 0,00089 | ACTC1 | 0,00027 | MYH11 | 0,00016 | LYN | 0,00008 | C1QA | 0,00002 |
| MTHFD 1 | 0,00086 | AP2B1 | 0,00027 | KRT2 | 0,00016 | WNT7A | 0,00008 | PAK4 | 0,00002 |
| SLC16A 1 | 0,00085 | NRP1 | 0,00026 | LTF | 0,00015 | LGALS1 | 0,00007 | ATP2B4 | 0,00002 |
| CYRIB | 0,00082 | CD248 | 0,00025 | GJA1 | 0,00015 | CSPG4 | 0,00007 | TIMP1 | 0,00002 |
| C1QTN F3 | 0,00078 | AXL | 0,00025 | SRI | 0,00015 | SLC16A7 | 0,00007 | PDCD6 | 0,00002 |
| EFNB1 | 0,00068 | ITGA5 | 0,00025 | STAT1 | 0,00014 | LRP1 | 0,00007 | PPP2R1A | 0,00002 |
| CLEC11 A | 0,00067 | DSP | 0,00025 | ARF6 | 0,00014 | DNAJA1 | 0,00007 | NOTCH1 | 0,00002 |
| FLNC | 0,00066 | LIPA | 0,00025 | RAB7A | 0,00013 | PDGFRA | 0,00007 | SERINC3 | 0,00002 |
| FHL1 | 0,00059 | YWHAE | 0,00025 | ITGA6 | 0,00013 | RHOA | 0,00007 | RAB11B | 0,00002 |
| RDX | 0,00057 | CYFIP1 | 0,00025 | PLG | 0,00013 | RASA3 | 0,00007 | MMP2 | 0,00002 |
| SLC44A 1 | 0,00056 | SLC7A5 | 0,00024 | GSTO1 | 0,00013 | SLC1A4 | 0,00007 | KRT16 | 0,00002 |
| CAPNS 1 | 0,00055 | CTSK | 0,00023 | RELN | 0,00013 | STC2 | 0,00007 | VAMP2 | 0,00002 |
| NEO1 | 0,00052 | IGF2 | 0,00023 | PLXNB2 | 0,00013 | CTNNA1 | 0,00006 | GSN | 0,00002 |
| ATP1A1 | 0,00049 | RAB1A | 0,00023 | CAMK2 A | 0,00013 | IGFBP2 | 0,00006 | ADH5 | 0,00002 |
| SLIT3 | 0,00049 | RPS3 | 0,00023 | RHOG | 0,00013 | F2R | 0,00006 | RAC1 | 0,00001 |
| PLAUR | 0,00049 | GNAI3 | 0,00022 | B2M | 0,00013 | IGF2R | 0,00006 | EPHB3 | 0,00001 |
| CAPN1 | 0,00048 | RHOC | 0,00022 | S100A1 1 | 0,00013 | NOTCH3 | 0,00006 | ITGAV | 0,00001 |
| RHOB | 0,00047 | C1R | 0,00022 | SGCD | 0,00012 | AHNAK | 0,00006 | FAP | 0,00001 |
| GREM1 | 0,00045 | ADAM9 | 0,00022 | TF | 0,00012 | CFI | 0,00006 | HGS | 0,00001 |
| PLOD3 | 0,00045 | ATP2B1 | 0,00022 | PLVAP | 0,00012 | GPRC5B | 0,00005 | SERPIND 1 | 0,00001 |
| CORO1 C | 0,00044 | VCAM1 | 0,00022 | PDCD10 | 0,00012 | CTNNB1 | 0,00005 | EIF5A | 0,00001 |
| NOTCH 2 | 0,00044 | CLIC1 | 0,00021 | HBD | 0,00012 | C1S | 0,00005 | CACNA2D 1 | 0,00001 |
| SERPIN F1 | 0,00044 | GNA13 | 0,00021 | NME2 | 0,00011 | C1QB | 0,00005 | MASP2 | 0,00001 |
| RAC2 | 0,00041 | F10 | 0,00021 | PLP2 | 0,00011 | ANXA6 | 0,00005 | | |
| TFRC | 0,00041 | C8B | 0,00021 | MIF | 0,00011 | HAPLN3 | 0,00005 | | |

Further, the presence of the most abundant proteins were verified by western blot. In particular, the abundance of PPP2CB, LTBP4, STX4, INHBA, GNAI2, ENG, and LOXL2 was quantified.

Besides, from the total of 228 proteins only expressed in EVs-THN, the assays were also to focus on the following 19 proteins by their role in cardiac tissue repair (ITGA1, ITGA3, ITGA4, ITGA6, ITGAV, ITGB5, RHOB, NOTCH2, NOTCH3, ANGPT1, RHOG, MYOF, WNT7A, PDGFRA, IGFBP2, TGFB1, HGF, TIMP1 and NOTCH 1). In addition, among the proteins that were expressed both in EVs derived from MSC_{T} and MSC_{THN}, the following were expressed more than 2 fold in EVs_{THN} (table 2):

**Table 2. Existing Proteins sobrexpressed in EVs_{THN}**

| **Protein** | **NSAF EVs-T** | **NSAF EVs-THN** | **Fold Change** |
|---|---|---|---|
| **ENO1** | 0,001119584 | 0,251730253 | 224,8427481 |
| **PXDN** | 6,80724E-05 | 0,000903944 | 13,27914893 |
| **ITGB3** | 2,46289E-05 | 0,00028942 | 11,75121141 |
| **MAMDC2** | 9,78417E-05 | 0,001110015 | 11,3450092 |
| **NID2** | 4,22901 E-05 | 0,000357556 | 8,454829735 |
| **HSPG2** | 0,000103573 | 0,000861752 | 8,320205692 |
| **KRT9** | 0,000173319 | 0,00143679 | 8,289870266 |
| **COL4A2** | 3,39655E-05 | 0,000218016 | 6,418740144 |
| **FBN1** | 0,000157136 | 0,000996993 | 6,344783677 |
| **SVEP1** | 1,08558E-05 | 6,41529E-05 | 5,909568625 |
| **LAMA4** | 0,000273339 | 0,001528638 | 5,592467273 |
| **COL3A1** | 0,00011446 | 0,000634261 | 5,541329493 |
| **ANGPTL2** | 0,000167998 | 0,000801398 | 4,770285939 |
| **LAMA2** | 5,37471 E-05 | 0,000256345 | 4,769472937 |
| **COL18A1** | 7,6533E-05 | 0,000358897 | 4,689445767 |
| **LAMB2** | 0,000249673 | 0,001104456 | 4,42360282 |
| **ADAM10** | 6,13025E-05 | 0,000254828 | 4,156885915 |
| **FGG** | 0,000171152 | 0,000690619 | 4,03511829 |
| **YWHAG** | 0,000156947 | 0,000566926 | 3,612213563 |
| **LAMB1** | 0,000421973 | 0,001341298 | 3,178634201 |
| **YWHAQ** | 0,000237342 | 0,00070264 | 2,960447764 |
| **VWF** | 3,44525E-05 | 0,000100497 | 2,916988329 |
| **HSP90AB1** | 0,000249743 | 0,000674717 | 2,701643391 |
| **EMILIN1** | 0,000375584 | 0,000960181 | 2,556504371 |
| **PDCD6IP** | 0,000329482 | 0,000824999 | 2,503923857 |
| **COL6A2** | 0,001374771 | 0,003379622 | 2,458315911 |
| **NID1** | 0,000326463 | 0,000795872 | 2,437863988 |
| **FBN2** | 0,000181024 | 0,000440842 | 2,435266607 |
| **SRPX** | 0,000433962 | 0,001049071 | 2,417427394 |
| **GPC1** | 0,000120071 | 0,00028018 | 2,333458676 |
| **LAMC1** | 0,000926843 | 0,002056278 | 2,218582343 |
| **FLNA** | 0,000136047 | 0,000299279 | 2,199815662 |
| **COL6A1** | 0,001885466 | 0,004051292 | 2,148695048 |
| **POSTN** | 0,00150315 | 0,003166185 | 2,106366995 |

Next, a microRNA sequencing of sEVs extracts from sEVs_{T} and sEVs_{THN} was performed. 128 miRNAs were identified exclusively in EV_{THN}. The results were filtered to obtain the most abundant miRNAs in sEVs_{THN}. A total of 36 miRNAs showed more than 1,000 counts (Table 3). Among them, the 9 miRNAs highly expressed were selected.

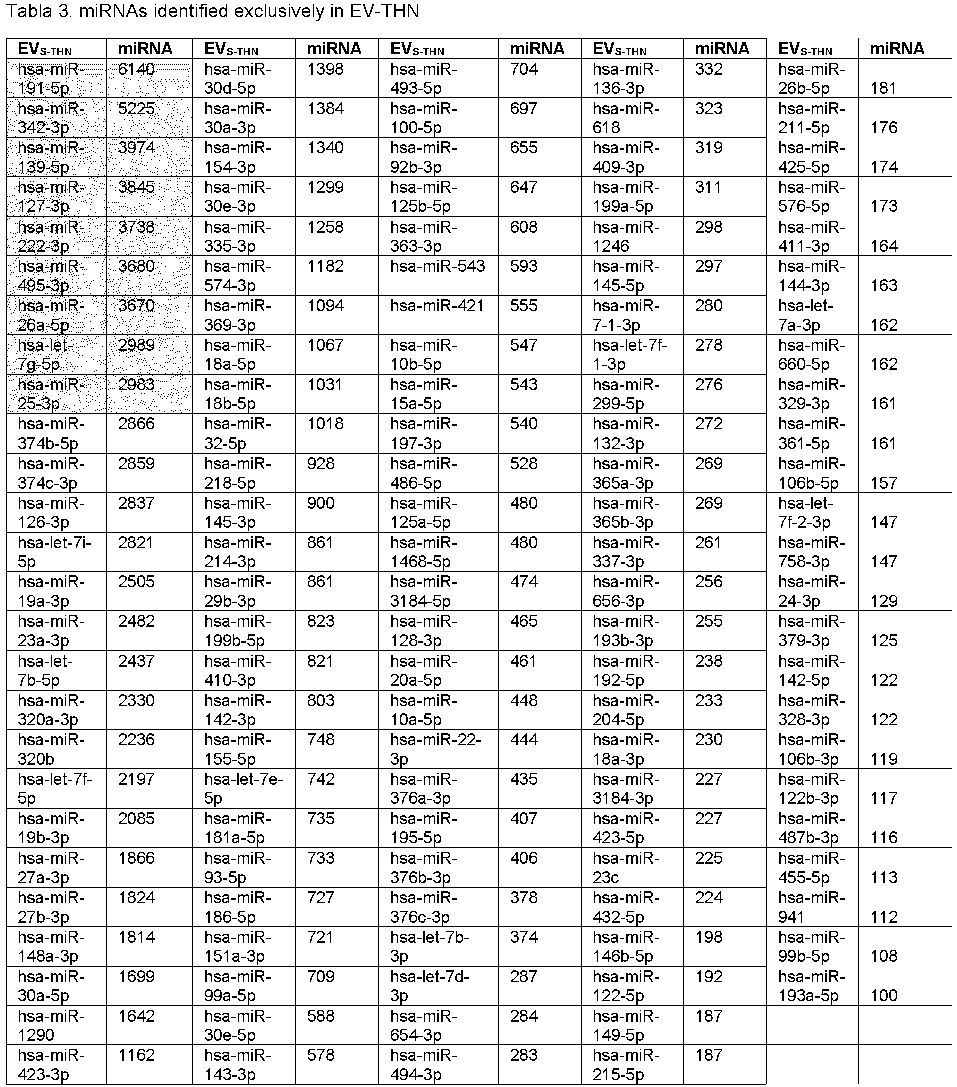

### 3. sEVsTHN reduce the expression of fibrosis markers and migration in stimulated cardiac fibroblasts

Next, the effect of these vesicles on human cardiac fibroblasts (HCF) stimulated to differentiate into myofibroblasts was tested. HCF were incubated in the stimulation medium for 24 hours and then treated with the different types of sEVs. The expression of genes related to the fibrosis process in HCF was analysed. Stimulation of HCF increased NOTCH1 expression (Figure 5). The NOTCH signalling pathway exerts an antagonistic function concerning the TGF-β signalling pathway. Consequently, this increase in NOTCH1 expression could be considered an inhibitory cellular mechanism. sEVs addition significantly reduce the increase in Notch due to stimulation, suggesting that sEVs are reducing the TGF-β-activated pathway.

Expression of α-smooth muscle actin (α-SMA; ACTA2) and fibroblast activation protein (FAP), typical markers of myofibroblasts, was also assessed. HCF stimulation led to a significant increase in ACTA2 expression while treatment with sEVs reduced ACTA2 levels, being specially pronounced in the treatment with sEVsTN and sEVsTHN. In the case of FAP, HCF stimulation did not lead to a significant increase in expression. In this case, sEVsT treatment did not lead to any decrease in expression. In contrast, sEVs_{THN} treatment reduced FAP expression, as did sEVsTN treatment (Figure 5A).

Expression of genes involved in the fibrotic process and cardiac remodelling such as collagen 1 (COL1A1), fibronectin (FN1), matrix metalloprotease 2 (MMP2) and bone morphogenic protein 1.3 (BMP1.3) was tested. COL1A1 expression increased after HCF stimulation but was only significantly reduced by sEVsTHN treatment. FN1 expression also increased after HCF stimulation, and although a trend towards reduction was observed with sEVsTN and sEVsTHN treatments, it was not significant. The same was true for MMP2, where the treatments produced a non-significant reduction in its expression. BMP1.3 expression was reduced after treatment with sEVsTN and sEVsTHN. WNT1 expression was analysed and found to be significantly increased after HCF stimulation. However, treatment with both TN-sEVs and sEVsTHN resulted in a drastic down-regulation of its expression (Figure 5A). Since the WNT pathway converges with that of TGF-β in the process of cardiac fibrosis, reducing its expression could have beneficial therapeutic implications.

In addition to gene expression, the effect of sEVs on the transition of HCF to myofibroblasts by immunocytochemistry was also studied. Representative images of each experimental condition are shown in Figure 5B. The fluorescent signal of α*-*SMA was quantified by measuring MFI per cell number (Figure 5C). The α*-*SMA fluorescent signal was barely observed in the control condition. However, the MFI was significantly elevated when HCF were stimulated. The MFI was slightly lower in cells treated with sEVsT than in stimulated cells.

Regarding the treatment of HCF with sEVsTN and sEVsTHN, a significant reduction of α*-*SMA MFI was observed compared to stimulated cells and also compared to sEVsT-treated cells.

In addition to the overproduction of ECM components, the fibrotic process is also characterised by the invasion of myofibroblasts from other parts of the damaged tissue. Therefore, the migration capacity of HCFs was tested by performing a scratch assay. Complete closure of wound area was observed in untreated stimulated HCF 24 hours after the start of the assay. Treatment with TN-sEVs reduced HCF migration. This reduction was even greater when HCFs were treated with sEVsTHN (Figure 5D and E).

The results obtained in this section indicate that MSC-T-derived sEVs have some intrinsic capacity to mitigate fibroblast activation and differentiation to myofibroblasts upon stimulation. Furthermore, overexpression of N1ICD and N1ICD- HIF1α by MSC-T enhances the therapeutic potential of sEVs in inhibiting the fibrotic process.

### 4. sEVs-THN protect CM in an in vitro model of ischemia and reduce hypertrophy markers after AngII stimulation

After assessing the impact of sEVs on fibroblasts, experiments using the murine myoblast line H9C2, which is widely recognised for its similarity to CM, were carried out. CM are cells that are highly sensitive to oxygen levels. When ischemic damage occurs, a necrotic zone is generated as a result of cell death of these cells due to oxidative stress. To test whether the different types of sEVs had anti-apoptotic potential, two viability assays were performed on H9C2 cells treated with sEVs and subjected to OGD for 7 hours. The metabolic activity of H9C2 was measured using a CCK-8 assay (Figure 6A) and plasma membrane integrity was assessed by quantifying the LDH enzyme activity present in the medium (Figure 6B). In both assays, an increase in cell viability was observed when cells were treated with sEVsTHN. In the CCK-8 assay, a significant increase in viability was also observed when cells were treated with TN-sEVs.

The impact of different types of sEVs on cell hypertrophy was assessed. Hypertrophy was induced in H9C2 cells by stimulation with angiotensin II (Ang II), followed by the addition of sEVs and a 24-hour incubation period. Ang II is known to be a potent inducer of cardiac hypertrophy and is widely used in in vitro and in vivo studies. After this period of Ang II exposure, the expression of three classic markers of cardiac hypertrophy were analysed: atrial natriuretic peptide (ANP), brain natriuretic peptide (BNP) and β-myosin heavy chain (β-MHC). The results showed that Ang II exposure caused a significant increase in the expression of all three markers (as shown in Figure 6C). Treatment with sEVs reduced the expression of all these markers, with the exception of ANP in the case of treatment with sEVsT, where no significant reduction in its expression was observed. Overall, all three types of sEVs proved to be highly effective in counteracting the effects of Ang II in inducing cell hypertrophy, suggesting therapeutic potential in the context of hypertrophy-related heart disease.

Once proven that sEVsTHN protected cells from OGD damage, the levels of ROS generated during OGD were examined. To this end, two assays following the previous protocol of keeping the cells in OGD for 7 hours with or without the addition of sEVs were performed. First, DCFH-DA staining was performed to quantify intracellular ROS production. As shown in Figures 6 D, the OGD protocol increased ROS levels, while the EV treatments decreased it, being significant in the case of TN-EVS and sEVs-THN. Of note the reduction in ROS levels induced by the sEVsTHN treatment was also significantly higher compared to the T-sEVs treated condition. Secondly, superoxide production in mitochondria was also assessed using MitoSOX staining. Mitochondria play a crucial role in cardiomyocytes and are one of the most affected cell parts under ischemic conditions. As seen in Figures 6E , exposure of the cells to the OGD protocol without treatment resulted in a significant increase in superoxide production in the mitochondria. T-sEVs treatment were able to reduce the generation of this mitochondrial superoxide. sEVsTHN treatment significantly decreased the amount of superoxide in mitochondria compared to untreated cells and T-sEVs treated cells. Furthermore, it is relevant to note that the levels of superoxide detected after sEVs-THN treatment were even lower than those observed in cells under normoxic conditions, indicating a potent antioxidant effect exerted by these EVs.

Overall, these results suggest an anti-apoptotic and antioxidant role of sEVs-THN on ischemic CM. The ability of -sEVsTHN to preserve cell viability and decrease ROS levels could have important therapeutic applications in the context of ischaemic heart disease, where preserving CM viability and function is essential to mitigate cardiac damage.

### 5. sEVsTHN increase angiogenesis in vitro

A key aspect in the repair of damaged cardiac tissue is the angiogenic capacity of endothelial cells present in the heart. To assess the pro-angiogenic potential of different types of sEVs, a functional tube-forming assay was performed on Matrigel with umbilical cord vein endothelial cells (HUVECs). Cells were placed in a hypoxia incubator for 9 hours with or without the addition of sEVs. In this way, the angiogenic potential of sEVs under conditions similar to those produced in ischemic heart disease was assessed (Figure 7A).

Results obtained, as shown in Figure 7B, indicate a significant increase in total tube length as well as total tubes in sEVsTHN-treated cells compared to untreated and sEVsT-treated cells. As for branch points, only sEVsTHN managed to significantly increase them. The same was true for total loops, where only sEVsTHN were able to produce a significant increase over the untreated condition.

These results suggest that HIF1α overexpression, together with N1ICD overexpression, is indeed a determinant for a more robust pro-angiogenic effect under hypoxic conditions. These findings support the hypothesis that sEVs enriched with N1ICD and HIF1α may represent a promising therapeutic tool in the context of cardiovascular diseases such as ischemic heart disease, where the formation of new blood vessels is essential for the regeneration of damaged tissue.

### 6. sEVs-THN enhances neoangiogenesis, reduces infarct size, and increases ejection fraction in a model of type 1 AMI.

After evaluating the in vitro effect of sEVsTHN, they were injected into mice subjected to permanent coronary artery ligation. A single dose of sEVs was administered intracardiac right after ischemia induction. Cardiac magnetic resonance was performed at 1, 7, and 21 days after coronary ligation. Our results pointed out that, mice treated with sEVsTHN displayed a more conserved systolic function (lower left ventricular end-systolic volume index and higher left ventricular end-diastolic volume index) and preserved cardiac structure (reduced infarct extension and enlarged infarct wall thickness) (Figures 8A-D).

Next, microscopic analysis of the hearts was executed to quantify the presence of collagen using Masson's trichromic staining. According to our results, the number of segments with interstitial fibrosis was reduced in animals undergoing sEVsTHN administration compared to saline (Figure 8E and 8F). In this line, left ventricular thickness, both globally and concretely within the fibrotic area, was preserved in animals undergoing sEVsTHN treatment (Figures 8G and 8H). Then, vascularization was also characterized via immunohistochemistry using anti-CD31 antibody (Figure 8I). The number of CD31-positive cells was significantly enlarged in mice treated with sEVsTHN compared with saline group, both in infarcted and remote regions (Figure 8J).

To further explore the influence of sEVsTHN on myocardial fibrosis, the mRNA expression of key genes implicated in cardiac fibrosis were determined using qRT-PCR. Concretely, the transcriptomic levels of col1a1 and col3a1, the two main subunits that constitute the post-infarction fibrotic scar, were significantly diminished in animals treated with EV after induction of coronary ischemia (Figures 8K and 8L). Similarly, mRNA levels of acta2 (myofibroblast marker) as well as ctgf and tgfb (cardiac fibrosis regulatory factors) were found to be significantly reduced in animals treated with this new biological product compared to mice treated with serum (Figures 9M-O).

### 7. sEVsTHN reduce myofibroblast and fibrosis in cardiac tissue in a model of type 1 AMI.

A strain of Postn (periostin) gene-targeted mice containing a tamoxifen-inducible Cre for cellular lineage-tracing analysis was previously generated (Onur Kanisicak et al. Genetic lineage tracing defines myofibroblast origin and function in the injured heart. Nat Commun. 2016 Jul 22:7:12260. doi: 10.1038/ncomms12260*).* This Postn allele identifies essentially all myofibroblasts within the heart and multiple other tissues. Using this animals, named Pst/Tmt, intramyocardically saline or sEVs-THN were transplanted after inducing myocardial infarction type 1. The results showed a drastic reduction of myofibroblasts accumulation in the heart of animals as visualized using IVIS spectrum and after perisotin quantification in heart sections (Figure 9A-C).

### 8. sEVs-THN reduce fibrosis and hypertrophy and increase vascular density in a type 2 myocardial infarction model by isoproterenol infusion

Once scrutinizing the beneficial effects of sEVsTHN in type 1 myocardial infarction, these data were corroborated on a murine model of type 2 myocardial infarction. The protocol used consisted of administering a daily dose of 150 mg/kg isoproterenol for 5 days, with injection of sEVs on the first and seventh day, followed by sacrifice of the animals after 21 days (Figura 10A).

To assess myocardial fibrosis, heart sections were stained with picrosirius red and the area of collagen was quantified. Representative sections from the different experimental groups can be seen in Figure 10B. Treatment of mice with sEVsTHN showed a non-significant reduction of the fibrotic area compared to the control group (Figure 10C).

Next, the effect of sEVs-THN on pathological cardiac hypertrophy caused by ISO administration was evaluated. For this purpose, heart sections were stained with WGA to mark cell membranes and facilitate quantification of the average cardiomyocyte cross-sectional area (Figure 10D). Treatment of mice with sEVsTHN drastically reduced the cardiomyocytes area with respect to the control and the T-sEVs treated group (Figure 10E).

Finally, cardiac microvascular density was analyzed, which is always associated with a better prognosis after heart damage. Angiogenesis was quantified using an antibody against CD31 (Figure 10F). The positive results obtained in terms of angiogenesis in vitro with sEVs-THN were replicated in the in vivo model. Mice treated with these vesicles showed higher angiogenesis compared to the control group and the T-sEVs treated group (Figure 10G).

### 9. sEVs-THN prevents infarct progression and reduces vascular obstruction in a pig model

Opening the occlusion using an angioplasty balloon represents the main clinical intervention for AMI patients, while the introduction of the cardioprotective drug before blood flux restoration can help to prevent reperfusion-induced injury and limit infarct size. To ratify the value of sEVsTHN in a more clinically relevant scenario, an angioplasty catheter that allowed the infusion of drugs before the opening of the occluded artery in an AMI-induced swine model of I/R-induced injury was used.

First, left ventricular infarct area (LV IA) was measured seven days post-I/R-induced injury by TTC staining and microvasculature obstruction (MVO) by thioflavine-S, as the combination of these techniques has been described as the most accurate method to determine infarcted myocardium (Figure 11A). Intracoronary administration of PBS (I/R group, n = 5) and sEVs-THN (I/R + sEVsTHN, n=1) was performed shortly before reperfusion. An angioplasty catheter designed by LVD Biotech Medical Services for the intracoronary infusion of different treatments was used. This procedure allowed the infusion of cardioprotective agents before blood flux restoration in the hope of preventing reperfusion induced injury, a procedure that could be easily translated for use in human patients. The representative images and measurements depicted in Figure 11B respectively, demonstrate that sEVsTHN infusion before I/R reduced LVIA as measured by TTC compared to treatment with PBS (24,41% vs 9,061% respectively). When sEVsTHN was infused after 45 min of coronary occlusion and shortly before reperfusion, a reduction in MVO compared to the PBS group was observed, as measured by thiflavine-S staining (11,75% vs 4,57% respectively, Figure 11C).

## Claims

1. A lipid nanoparticle (LNP), comprising:
- a protein composition comprising at least one of the following proteins,
• PPP2CB,
• LTBP4,
• STX4,
• INHBA,
• GNAI2,
• ENG, and
• LOXL2,
- and a miRNA profile comprise at least one of the following miRNAs:
• hsa-miR-191-5p,
• hsa-miR-342-3p,
• hsa-miR-139-5p,
• hsa-miR-127-3p,
• hsa-miR-222-3p,
• hsa-miR-495-3p,
• hsa-miR-26a-5p,
• hsa-let-7g-5p, and
• hsa-miR-25-3p
wherein the LNP has a size between 50 and 150 nm.

2. LNP according to claim 1, further comprising at least one of the following proteins: ITGA1, ITGA3, ITGA4, ITGA6, ITGAV, ITGB5, RHOB, NOTCH2, NOTCH3, ANGPT1, RHOG, MYOF, WNT7A, PDGFRA, IGFBP2, TGFB1, HGF, TIMP1 and NOTCH1.

3. LNP according to claim 1 or 2 wherein the expression levels of at least one of the following proteins are increased by at least two fold: ENO1, PXDN, ITGB3, MAMDC2, NID2, HSPG2, KRT9, COL4A2, FBN1, SVEP1, LAMA4, COL3A1, ANGPTL2, LAMA2, COL18A1, LAMB2, ADAM10, FGG, YWHAG, LAMb1, YWHAQ, VWF, HSP90AB1, EMILIN1, PDCD6IP, COL6A2, NID1, FBN2, SRPX, GPC1, LAMC1, FLNA, COL6A1 and POSTN.

4. LNP according to claim 1 wherein said nanoparticle is selected from the group comprising:
a) LNP obtained from a natural source, and
b) synthetic LNP obtained from chemical or biological synthesis methods.

5. LNP according to claim 4, wherein the LNP is an EV derived from modified parental cells.

6. LNP according to claim 5 wherein parental cells are mesenchymal stem cells (MSCs).

7. LNP according to claim 6 wherein the MSCs are genetically modified to overexpress hTERT, HIF1α and N1ICD (MSCs-THN).

8. LNP according to claim 4, wherein the LNP is a synthetic liposome.

9. Use of the LNP, according to any one of claims 1 to 8, as a nanocarrier, nanovector and/or nanocapsule for the release and/or encapsulation of molecules.

10. Pharmaceutical or therapeutic composition comprising one or more lipid nanoparticles (LNPs) according to any one of claims 1 to 8.

11. The LNP according to any one of claims 1 to 8, or the composition, according to claim 10, for use in the treatment of ischemic heart diseases.

12. The LNP or the composition for use, according to claim 11, wherein the ischemic heart disease is myocardial infarction.

13. The LNP according to any one of claims 1 to 8 or the composition according to claim 10 for use in a method of regenerating cardiac tissue in an individual having damaged cardiac tissue.

14. The LNP or the composition for use, according to claim 13, wherein the damaged cardiac tissue was damaged by ischemia.

15. Method for obtaining EVs isolated from MSCs genetically modified to overexpress HIF1α, hTERT and N1ICD, according to claim 7, comprising the steps of:
a. Immortalizing a culture of MSCs by overexpressing the human telomerase enzyme (hTERT) by lentiviral vector, obtaining MSC-T,
b. Expand the MSCs-T to a required quantity,
c. Overexpressing HIF1α and N1ICD in the immortalized MSCs-T, by lentiviral infection, obtaining MSCs-T-HIF1α-N1ICD (MSCs-THN), wherein the overexpression of N1ICD is induced by an inducible promotor, and
d. Isolating the EVs released by the MSCs-THN to the culture medium.
